# EUROPEAN PATENT SPECIFICATION

(11) **EP 1 244 805 B1**
(45) Date of publication and mention of the grant of the patent: **22.02.2006**
(21) Application number: 00981481.5
(22) Date of filing: 12.12.2000
(51) Int. Cl.: C12N 15/88, C12N 15/34, C07K 14/075, A61K 47/48

(54) **VIRAL CORE PROTEIN-CATIONIC LIPID-NUCLEIC ACID-DELIVERY COMPLEXES**
VIRALE KERNPROTEINE-KATIONISCHE LIPID-NUKLEINSÄURE-VERABREICHUNGKOMPLEXE
COMPLEXES D'ADMINISTRATION A BASE D'ACIDES NUCLEIQUES-LIPIDE CATIONIQUE-PROTEINE NOYAU VIRALE

(30) Priority: 23.12.1999 GB 9930533
(43) Date of publication of application: 02.10.2002
(60) Divisional application: 04077588.4
(73) Proprietor: MITSUBISHI CHEMICAL CORPORATION, Tokyo 108-0014 (JP)
(72) Inventor: TAGAWA, Toshiaki Mitsubishi-Tokyo Pharmaceuticals, Tokyo 103-8405 (JP); MILLER, A. D. Imper. College Genetic Therapies C., South Kensington, London SW7 (GB); PEROUZEL, E. Imperial College Genetic Therapies C., South Kensington, London SW7 (GB); MURRAY, Karl University of California-Davis, Davis, CA 95916 (US); MANVELL, Michelle Department of Gene Therapy, London SW3 6LR (GB); ALTON, Eric Department of Gene Therapy, London SW3 6LR (GB); MATTHEWS, David University of Leeds, Leeds LS9 7TF (GB); RUSSELL, Willie School of Biomedical Sciences, Fife KY16 9ST (GB)
(74) Representative: Alcock, David
(86) International application number: PCT/GB2000/004767
(87) International publication number: WO 2001/048233

(56) References cited:
- WO-A-96/40961
- WO-A-98/40502
- FASBENDER,A., ZABNER, J., CHILLON, M., MONINGER, T.O., PUGA, A.P., DAVIDSON, B.L., WELSH, J.: "complexes of adenovirus with polycationic polymers and cationic lipids increase the efficiency of gene transfer in vitro and in vivo" JOURNAL OF BIOLOGICAL CHEMISTRY, vol. 272, no. 10, 7 March 1997 (1997-03-07), pages 6479-6489, XP002165480
- MRSNY, R.J.: "A SURVEY OF THE RECENT PATENT LITERATUR ON THE DELIVERY OF GENES AND OLIGONUCLEOTIDES" JOURNAL FO DRUG TARGETING, vol. 7, no. 1, July 1999 (1999-07), pages 1-10, XP000985806

## Description

### Field of the invention

The present invention relates to cationic lipid/protein/nucleic acid complexes comprising Mu1 Adenoviral packaging proteins and their use in the efficient delivery of nucleic acids to cells, such as neuronal cells.

### Background to the invention

Promising advances in non-viral gene transfer have been made as a result of the production of synthetic liposomes formulated with cationic lipids that are able to transfect cells. However few of these complexes have been examined for their ability to efficiently transfer DNA into CNS cells and to obtain expression of a transgene. The ability to transfect neuronal cells efficiently and safely could provide a powerful tool for the elucidation of neuronal function and may lead to novel treatments for neurological disorders.

Unfortunately, gene therapy for the CNS has been hampered by the lack of efficient means for transducing postmitotic neurons. Most studies have utilized viral vectors for gene delivery. However, many viral vectors are plagued by problems of immunity and cytotoxicity and are not easily manipulated by non-virologists ¹⁻³. Non-viral vectors are now emerging as an alternative method of cellular transduction. The most promising advances in non-viral gene transfer have been in the production of synthetic liposomes formulated with cationic lipids (cytofectins) able to transfect cells. Such cationic liposomes are relatively easy to use, have a broad applicability and lack cytotoxicity ⁴.

Novel cationic liposome formulations are constantly being developed ⁵. However, few of these complexes have been examined for their ability to efficiently transduce cells within the CNS ⁶⁻⁹. Cationic liposomes act via electrostatic interactions with negatively charged DNA and subsequently with cellular membranes where they are taken across the cell membrane by a process of slow endocytosis ^{6, 10, 11}. They are frequently formulated using the neutral lipid dioleoyl-L-α-phosphatidylethanolamine (DOPE), which is extremely efficient at endosomal buffering and disruption ^{8, 12}. From the perinuclear space transfected genetic material is released from the liposome complex, transported to the nucleus and expressed. To date only liposomes formulated from N-[1-(2,3-dioleyloxy)propyl]-N,N,N trimethyl ammonium chloride (DOTMA) and DOPE, have been shown to mediate successful transfection in the CNS ¹³⁻¹⁶. To be useful for gene therapy liposome complexes capable of transfecting CNS cells with high efficiency are needed.

A major limitation in non-viral mediated gene transfer is the formation of large aggregated molecules during the generation of liposome:DNA complexes ⁵. These large aggregates may reduce the efficiency of transfection possibly by limiting endocytosis of the complexes. One approach to circumvent this is to reduce the size of DNA molecules via DNA condensation prior to complex formation. Pre-condensation of DNA produces smaller complexes and improved transfection efficiencies ¹⁷⁻²³. Various polycations have been identified which are efficient at improving liposome-mediated transfections. Of these, poly-L-lysine and protamine have produced the most dramatic results enabling increases of over 30 fold compared to complexes without pre-condensation in a variety of non-neuronal cell lines ^{17,21}.

Protamine sulphate is particularly good at enhancing liposomal transfection. Protamine is a naturally occurring polycation found in the head of spermatozoa. The role of protamine is to condense DNA in sperm and aid in its transfer to the egg nucleus. The nuclear targeting property of protamine makes it particularly attractive for gene transfer. Also, unlike the synthetic poly-L-lysine, which has a range of large molecular weights (18000-19200 Da), protamine is naturally occurring, smaller and more uniform in size (4000-4250 Da). These qualities mean there is less chance for immunogenic responses in the target tissue and the condensation is easier to control. Other naturally occurring DNA condensing proteins have also been used to enhance cationic liposome mediated DNA transfer. Fritz et al, ²² achieved approximately 30 fold increases in lipofection using a recombinant human H1 histone protein incorporating a nuclear localization signal (nls-H1). Also, the non-histone chromosomal high mobility group 1,2 protein has been shown to improve lipofection and is used routinely in the HVJ-liposome method ^{20, 24}.

### Summary of the invention

We have examined viral-DNA associated proteins for their ability to improve liposome based gene transfer. In particular we have compared the viral-coded synthetic peptide Mu1 and recombinant Vp1 protein of adenovirus and polyomavirus respectively. Mu1 may play a role in adenoviral chromosome condensation while VP1 is the only structural protein of polyomavirus to exhibit DNA binding activity ²⁵⁻²⁷. Vp1, but not Mu1 contains an embedded classical nuclear localization signal (NLS) similar to that found in HMG-1,2 and nls-H1²⁶. We found that Mu1, but not Vp1, significantly improved cationic liposome mediated gene transfer in cells derived from the nervous system and kidney. We also found that Mu1 enhancement was greater in differentiated cells indicating the possible usefulness of this approach for neuronal cells *in vivo*.

These findings have implications for experimental and therapeutic uses of liposome-mediated delivery of DNA to CNS cells.

Accordingly, the present invention provides a non-viral nucleic acid delivery vector comprising a condensed polypeptide/ nucleic acid complex and a cationic lipid, wherein the complex comprises
(a) a nucleic acid sequence of interest (NOI); and
(b) one or more Mu1 Adenoviral polypeptides, or homologous polypeptides with at least 60 % identity thereto, said polypeptides or said homologues thereof being (i) capable of binding to the NOI; and (ii) capable of condensing the NOI; and wherein the NOI is heterologous to the one or more polypeptides.

The term "heterologous to the polypeptide" means that viral NOIs that naturally occur in combination with the viral packaging polypeptide are excluded.

In a preferred embodiment, the vector further comprises a polypeptide comprising a nuclear localisation sequence (NLS). More preferably, the polypeptide comprising a nuclear localisation sequence (NLS) is adenoviral pV or a derivative thereof.

The present invention also provides a condensed polypeptide/nucleic acid complex comprising a cationic lipid, a polypeptide component and a nucleic acid component, for use in delivering the nucleic acid component to a nucleus of a eukaryotic cell, wherein
(i) the polypeptide component is a Mu1 Adenoviral polypeptide, or a homologous polypeptide with at lest 60 % identity thereto;
(ii) the polypeptide component or said homologue thereof is capable of binding to the NOI; and
(iii) the polypeptide component or said homologue thereof is capable of condensing the NOI;

and wherein the nucleic acid is heterologous to the polypeptide.

In a preferred embodiment, the complex further comprises a polypeptide comprising a nuclear localisation sequence (NLS). More preferably, the polypeptide comprising a nuclear localisation sequence (NLS) is adenoviral pV or a derivative thereof.

The present invention also provides a method of producing a non-viral nucleic acid delivery vector comprising a cationic lipid and a condensed polypeptidelnucleic acid complex, which method comprises
(a) contacting a nucleic acid sequence of interest (NOI) with a Mu1 Adenoviral polypeptide or a homologous polypeptide with at least 60 % identity thereto thereof, said polypeptide component or said homologue thereof being (i) capable of binding to the NOI; and (ii) capable of condensing the NOI; and wherein the NOI is heterologous to the polypeptide; and
(b) contacting the nucleic acid/polypeptide complex thus formed with a cationic lipid.

The present invention further provides a method of introducing a nucleic acid sequence of interest (NOI) into a eukaryotic cell which method comprises contacting the cell with a complex of the invention wherein the complex comprises the NOI. Preferably the cell is a neuronal, cancer or epithelial cell.

In an alternative embodiment, a viral nucleic acid nuclear localisation/delivery polypeptide may be used in addition to a Mu1 Adenoviral polypeptide. Indeed, some viral polypeptides combine both functions.

### Detailed description of the invention

Although in general the techniques mentioned herein are well known in the art, reference may be made in particular to Sambrook *et al.,* Molecular Cloning, A Laboratory Manual (1989) and Ausubel *et al.,* Short Protocols in Molecular Biology (1999) 4^{th} Ed, John Wiley & Sons, Inc.

### A. Polypeptide components

### 1. Viral nucleic acid packaging polypeptides

The term "viral nucleic acid packaging polypeptides" typically includes polypeptides encoded by viral genomes that occur naturally in viral particles where their function is to package, in particular condense, and deliver into the nucleus the nucleic acids constituting the viral genome into the virion.

The viral nucleic acid packaging polypeptide for use in the present invention is the Mu1 Adenoviral polypeptide shown immediately below as SEQ I.D. No. 1.

NH₂-Met-Arg-Arg-Ala-His-His-Arg-Arg-Arg-Arg-Ala-Ser-His-Arg-Arg-Met-Arg-Gly-
Gly-OH (SEQ I.D. No. 1).

A Mu1 Adenoviral nucleic acid packaging polypeptide for use in the present invention is capable of binding to nucleic acids, typically in a non-specific manner, preferably causing condensation of the nucleic acid. It is generally preferred that the condensed NOI has a size of equal to or less than 200 nm, such as from 50 to 200 nm, for optimal efficiency of delivery to a target cell.

The ability of Mu1 Adenoviral polypeptides to bind to nucleic acids may be determined *in vitro* using techniques such as gel electrophoresis including gel retardation assays (see materials and methods section and results section) and electrophoretic band shift mobility assays, ethidium bromide exclusion assays and affinity chromatography (for example using single- or double-stranded DNA cellulose).

The ability of Mu1 Adenoviral polypeptides to condense nucleic acids may be determined by, for example, circular dichroism (CD) spectroscopy (see, for example, Sato and Hosokawa, 1984, J. Biol. Chem. 95: 1031-1039).

Generally the Mu1 Adenoviral polypeptides, or homologues thereof with at least 60 % identity thereto, will comprise a number of positively charged amino acid residues at physiological pH (such as pH 7.4). Preferably the overall net charge on the Mu1 Adenoviral polypeptide is positive at physiological pH. In particular, it is preferred that the charge : amino acid ratio is at least +0.3, preferably at least +0.4, +0.5 or +0.6.

It is preferred that the Mu1 Adenoviral polypeptides, or homologues thereof with at least 60 % identity thereto comprise arginine residues rather than lysine residues or a mixture of both. It is also particularly preferred that the Mu1 Adenoviral polypeptides,or homologues thereof with at least 60 % identity thereto comprise one or more histidine residues, preferably two or more histidine residues. In addition, the Mu1 Adenoviral polypeptides, or homologues thereof with at least 60 % identity thereto, will typically comprise a number of highly hydrophobic residues, such as alanine, for example two or more hydrophobic residues.

It will be understood that amino acid sequences for use in the invention are not limited to naturally occurring Mu1 Adenoviral nucleic acid packaging polypeptides but also include homologous sequences with at least 60% identity thereto obtained from any source, for example related viral/bacterial proteins, cellular homologues and synthetic peptides.

In the context of the present invention, a homologous sequence is taken to include an amino acid sequence which is at least 60, 70, 80 or 90% identical, preferably at least 95 or 98% identical at the amino acid level over at least 10 preferably at least 20, 30, 40 or 50 amino acids with the Mu1 sequence shown as SEQ I.D. No. 1. In particular, homology should typically be considered with respect to those regions of the sequence known to be essential for nucleic acid binding rather than non-essential neighbouring sequences. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux *et al.,* 1984, Nucleic Acids Research 12:387). Examples of other software than can perform sequence comparisons include, but are not limited to, the BLAST package (see Ausubel *et al.,* 1999 *ibid* - Chapter 18), FASTA (Atschul *et al.,* 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The term "derivative" in relation to the Adenoviral pV amino acid sequences used in the present invention includes any substitution of, variation of, modification of, replacement of, deletion of or addition of one (or more) amino acids from or to the sequence providing the resultant amino acid sequence has nucleic acid binding and condensation activity, preferably having at least the same activity as the unmodified polypeptides.

Mu1 Adenoviral polypeptides may be modified for use in the present invention. Typically, modifications are made that maintain the nucleic acid binding and condensation properties of the sequence. Amino acid substitutions may be made, for example from 1, 2 or 3 to 10, 20 or 30 substitutions provided that the modified sequence retains nucleic acid binding and condensation properties. Amino acid substitutions may include the use of non-naturally occurring analogues, for example to increase blood plasma half-life of a therapeutically administered polypeptide.

In particular, it may be desirable to make amino acid substitutions to increase the net positive charge, at physiological pH, of a naturally occurring Mu1 Adenoviral packaging polypeptide. Positively charged amino acids include arginine, lysine and histidine. Arginine is the most highly charged of the naturally occurring amino acids and is particularly preferred.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Conservative substitutions may be made, for example according to the Table above. Amino acids in the same block in the second column and preferably in the same line in the third column may be substituted for each other:
Polypeptides for use in the invention may be made by recombinant means, for example as described below. However they may also be made by synthetic means using techniques well known to skilled persons such as solid phase synthesis. Polypeptides for use in the invention may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein partner will not hinder the biological activity of the protein of interest sequence.
Polypeptides for use in the invention may be in a substantially isolated form. It will be understood that the polypeptides may be mixed with carriers or diluents which will not interfere with the intended purpose of the polypeptides and still be regarded as substantially isolated. The polypeptides may also be in a substantially purified form, in which case generally more than 90%, e.g. 95%, 98% or 99% of the protein in the preparation comprises polypeptides for use in the invention.

### 2. Polypeptides comprising nuclear localisation sequences

In a preferred embodiment, the delivery vector/complex of the invention further comprises a polypeptide comprising a nuclear localisation sequence (NLS). In general, NLSs are well known in the art (see, for example, Dingwall and Laskey, 1991, Trends. Biochem. Sci. 16: 478-481). However, it is particularly preferred to use the NLS of adenovirus core protein pV. The NLS of pV has the sequence RPRRRATTRRRTTTGTRRRRRRR (SEQ I.D. No.2) corresponding to amino acids 315-337 (D. Matthews, submitted.) A further NLS is present in the N-terminus (KPRKLKRVKKKKK - SEQ I.D. No. 3), although the C-terminal NLS is preferred.

The NLS may be present on a separate polypeptide molecule to the packaging polypeptide or as part of the same polypeptide chain, for example in a fusion protein.

### B. Nucleic acid sequences of interest

Nucleic acid sequences of interest (NOIs) intended to be delivered to cells using the delivery vector or complex of the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of the NOIs.

The NOI typically comprises a heterologous gene. The term "heterologous gene" encompasses any gene, The heterologous gene may be any allelic variant of a wild-type gene, or it may be a mutant gene. The term "gene" is intended to cover nucleic acid sequences which are capable of being at least transcribed. Thus, sequences encoding mRNA, tRNA and rRNA, as well as antisense constructs, are included within this definition. Nucleic acids may be, for example, ribonucleic acid (RNA) or deoxyribonucleic acid (DNA) or analogues thereof. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements.

The transcribed sequence of the heterologous gene is preferably operably linked to a control sequence permitting expression of the heterologous gene in mammalian cells, preferably neuronal cells, such as cells of the central and peripheral nervous system, cancer or epithelial cells. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence.

The control sequence comprises a promoter allowing expression of the heterologous gene and a signal for termination of transcription. The promoter is selected from promoters which are functional in mammalian, preferably human cells. The promoter may be derived from promoter sequences of eukaryotic genes. For example, it may be a promoter derived from the genome of a cell in which expression of the heterologous gene is to occur, preferably a cell of the mammalian central or peripheral nervous system. With respect to eukaryotic promoters, they may be promoters that function in a ubiquitous manner (such as promoters of β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter or promoters of herpes virus genes.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above. Furthermore, the use of locus control regions (LCRs) may be desirable.

The heterologous gene will typically encode a polypeptide of therapeutic use. In accordance with the present invention, suitable NOI sequences include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives therof (such as with an associated reporter group).

Examples of polypeptides of therapeutic use include neurotrophic factors such as nerve growth factor (NGF), ciliary neurotrophic factor (CNTF), brain-derived neurotrophic factor (BNTF) and neurotrophins (such as NT-3, NT-4/5) which have potential as therapeutic agents for the treatment of neurological disorders such as Parkinson's disease.

Suitable NOIs for use in the present invention in the treatment or prophylaxis of cancer include NOIs encoding proteins which: destroy the target cell (for example a ribosomal toxin), act as: tumour suppressors (such as wild-type p53); activators of anti-tumour immune mechanisms (such as cytokines, co-stimulatory molecules and immunoglobulins); inhibitors of angiogenesis; or which provide enhanced drug sensitivity (such as pro-drug activation enzymes); indirectly stimulate destruction of target cell by natural effector cells (for example, strong antigen to stimulate the immune system or convert a precursor substance to a toxic substance which destroys the target cell (for example a prodrug activating enzyme). Encoded proteins could also destroy bystander tumour cells (for example with secreted antitumour antibody-ribosomal toxin fusion protein), indirectly stimulated destruction of bystander tumour cells (for example cytokines to stimulate the immune system or procoagulant proteins causing local vascular occlusion) or convert a precursor substance to a toxic substance which destroys bystander tumour cells (eg an enzyme which activates a prodrug to a diffusible drug).

NOI(s) may be used which encode antisense transcripts or ribozymes which interfere with the expression of cellular or pathogen genes, for example, with expression of cellular genes for tumour persistence (for example against aberrant *myc* transcripts in Burkitts lymphoma or against *bcr-abl* transcripts in chronic myeloid leukemia. The use' of combinations of such NOIs is also envisaged.

Instead of, or as well as, being selectively expressed in target tissues, the NOI or NOIs may encode a pro-drug activation enzyme or enzymes which have no significant effect or no deleterious effect until the individual is treated with one or more pro-drugs upon which the enzyme or enzymes act. In the presence of the active NOI, treatment of an individual with the appropriate pro-drug leads to enhanced reduction in tumour growth or survival.

A pro-drug activating enzyme may be delivered to a tumour site for the treatment of a cancer. In each case, a suitable pro-drug is used in the treatment of the patient in combination with the appropriate pro-drug activating enzyme. An appropriate pro-drug is administered in conjunction with the vector. Examples of pro-drugs include: etoposide phosphate (with alkaline phosphatase); 5-fluorocytosine (with cytosine deaminase); doxorubicin-N-p-hydroxyphenoxyacetamide (with penicillin-V-amidase); para-N-bis(2-chloroethyl) aminobenzoyl glutamate (with carboxypeptidase G2); cephalosporin nitrogen mustard carbamates (with β-lactamase); SR4233 (with P450 Reducase); ganciclovir (with HSV thymidine kinase); mustard pro-drugs with nitroreductase and cyclophosphamide (with P450).

Examples of suitable pro-drug activation enzymes for use in the invention include a thymidine phosphorylase which activates the 5-fluoro-uracil pro-drugs capcetabine and furtulon; thymidine kinase from herpes simplex virus which activates ganciclovir; a cytochrome P450 which activates a pro-drug such as cyclophosphamide to a DNA damaging agent; and cytosine deaminase which activates 5-fluorocytosine. Preferably, an enzyme of human origin is used

NOIs may also encode antigenic polypeptides for use as vaccines. Preferably such antigenic polypeptides are derived from pathogenic organisms, for example bacteria or viruses. Examples of such antigenic polypeptides include hepatitis C virus antigens, hepatitis B surface or core antigens, HIV antigens, pertussis toxin, cholera toxin or diphtheria toxin.

NOIs may also include marker genes (for example encoding β-galactosidase or green fluorescent protein) or genes whose products regulate the expression of other genes (for example, transcriptional regulatory factors).

Where a disease is caused by a defective gene, NOIs may be admistered that encode a fully functional allele of the gene, such as in the case of cystic fibrosis. The molecular basis for a variety of genetic disorders has been identified and wild type functional sequences cloned. It may be desirable to include in the NOI flanking sequences to the therapeutic gene that are homologous to the corresponding flanking sequences in the genome to allow for replacement of the defective gene by homologous recombination.

Gene therapy and other therapeutic applications may well require the administration of multiple genes. The expression of multiple genes may be advantageous for the treatment of a variety of conditions. Since there is no limitation in the size of NOI that may be incorporated into a delivery vector or complex of the invention, it should be possible to target cells with multiple genes simultaneously.

### C. Cationic lipids

A variety of cationic lipids is known in the art - see for example WO95/02698, the disclosure of which is herein incorporated by reference, some of which is reproduced below. Example structures of cationic lipids useful in this invention are provided in Table 1 of WO95/02698. Generally, any cationic lipid, either monovalent or polyvalent, can be used in the compositions and methods of this invention. Polyvalent cationic lipids are generally preferred. Cationic lipids include saturated and unsaturated alkyl and alicyclic ethers and esters of amines, amides or derivatives thereof. Straight-chain and branched alkyl and alkene groups of cationic lipids can contain from 1 to about 25 carbon atoms. Preferred straight-chain or branched alkyl or alkene groups have six or more carbon atoms. Alicyclic groups can contain from about 6 to 30 carbon atoms. Preferred alicyclic groups include cholesterol and other steroid groups. Cationic lipids can be prepared with a variety of counterions (anions) including among others: chloride, bromide, iodide, fluoride, acetate, trifluoroacetate, sulfate, nitrite, and nitrate.

A well-known cationic lipid is N-[1-(2,3-dioleyloxy)propyl]-N,N,N-trimethylammonium chloride (DOTMA).

DOTMA and the analogous diester DOTAP (1,2-bis(oleoyloxy)-3 (trimethylammonium) propane), are commercially available. Additional cationic lipids structurally related to DOTMA are described in U.S. Patent 4,897,355, which is herein incorporated by reference.

Another useful group of cationic lipids related to DOTMA and DOTAP are commonly called DORI-ethers or DORI-esters. DORI lipids differ from DOTMA and DOTAP in that one of the methyl groups of the trimethylammonium group is replaced with a hydroxyethyl group. The oleoyl groups of DORI lipids can be replaced with other alkyl or alkene groups, such as palmitoyl or stearoyl groups. The hydroxyl group of the DORI-type lipids can be used as a site for further functionalization, for example for esterification to amines, like carboxyspennine.

Additional cationic lipids which can be employed in the delivery vectors or complexes of this invention include those described in WO91/15501as useful for the transfection of cells.

Cationic sterol derivatives, like 3β[N-(N',N'- dimethylaminoethane)carbamoyl] cholesterol (DC-Chol) in which cholesterol is linked to a trialkyammonium group, can also be employed in the present invention. DC-Chol is reported to provide more efficient transfection and lower toxicity than DOTMA-containing liposomes for some cell lines. DC-Chol polyamine variants such as those described in WO97/45442 may also be used.

Polycationic lipids containing carboxyspermine are also useful in the delivery vectors or complexes of this invention. EP-A-304111 describes carboxyspermine containing cationic lipids including 5-carboxyspermylglycine dioctadecyl-amide (DOGS) and dipalmitoylphosphatidylethanolamine 5-carboxyspermylamide (DPPES). Additional cationic lipids can be obtained by replacing the octadecyl and palmitoyl groups of DOGS and DPPES, respectively, with other alkyl or alkene groups.

In the delivery vectors or complexes of the invention cationic lipids can optionally be combined with non-cationic co-lipids, preferably neutral lipids, to form liposomes or lipid aggregates. Neutral lipids useful in this invention include, among many others: lecithins; phosphatidylethanolamines, such as DOPE (dioleoyl phosphatidylethanolamine), POPE (palmitoyloleoylphosphatidylethanolamine) and DSPE (distearoylphosphatidylethanol amine); phosphatidylcholine; phosphatidylcholines, such as DOPC (dioleoyl phosphatidylcholine), DPPC (dipalmitoylphosphatidylcholine) POPC (palmitoyloleoyl phosphatidylcholine) and DSPC (distearoylphosphatidylcholine); phosphatidylglycerol; phospha- tidylglycerols, such as DOPG (dioleoylphosphatidylglycerol), DPPG (dipalmitoylphosphatidylglycerol), and DSPG (distearoylphosphatidylglyceiol); phosphatidylserines, such as dioleoyl- or dipalmitoylphospatidylserine; diphospha tidylglycerols; fatty acid esters; glycerol esters; sphingolipids; cardolipin; cerebrosides; and ceramides; and mixtures thereof. Neutral lipids also include cholesterol and other 3DOH-sterols.

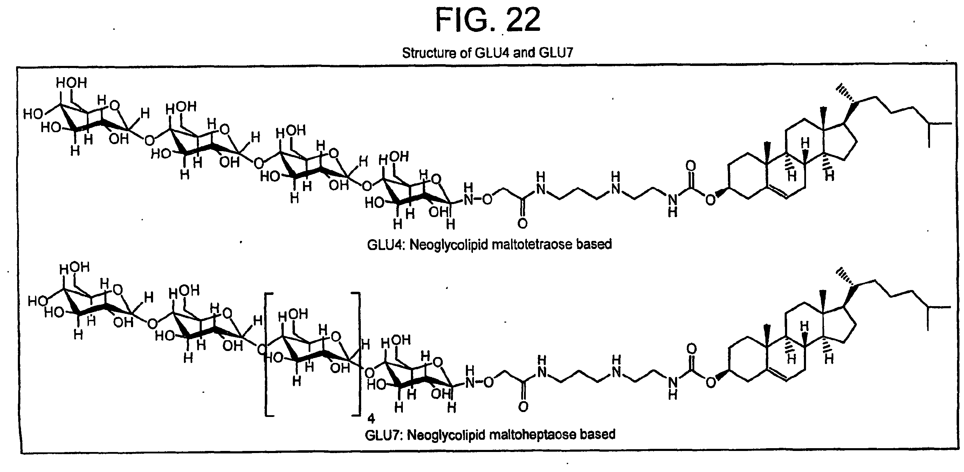

Moreover in the delivery vector or complexes of the invention one or more amphiphilic compounds can optionally be incorporated in order to modify its surface property. Amphiphilic compounds useful in this invention include, among many others; neoglycolipids such as GLU4 and GLU7 shown in Figure 22, polyethyleneglycol lipids such as N-(ω-methoxy(polyoxyethylene)oxycarbonyl)-phosphatidylethanolamine, N-monomethoxy(polyoxyethylene)succinylphosphatidylethanol-amine and polyoxyethylene cholesteryl ether; nonionic detergents such as alkyl, glycosides, alkyl methyl glucamides, sucrose esters, alkyl polyglycerol ethers, alkyl polyoxyethylene ethers and alkyl sorbitan oxyethylene ethers and steroidal oxyethylene ethers; block copolymers such as polyoxyethylene polyoxypropylene block copolymers.

In one aspect the cationic lipid of the present invention is modified with a sugar moiety or a polyethylene glycol (PEG) moiety. In a further aspect the complex of the invention further comprises a compound capable of acting as a cationic lipid, the compound comprising a cholesterol group having linked thereto via an amine group, a sugar moiety or a polyethylene glycol moiety. As demonstrated in the Examples we have found such sugar/PEG modified cationic lipids to be particularly advantageous. Thus in a further aspect the present invention provides a compound capable of acting as a cationic lipid, the compound comprising a cholesterol group having linked thereto via an amine group, a sugar moiety or a polyethylene glycol moiety. Preferably the compound comprises from 1 to 7 sugar moieties or a polyethylene glycol moieties. The compound may comprise a mixture of sugar moieties and polyethylene glycol moieties. Preferably the sugar moiety is or is derived from glucose or D-glucose.

### D. Cationic lipid/NOI/packaging polypeptide complexes

A delivery vector/complex of the present invention is typically made by firstly contacting a Mu1 Adenoviral packaging polypeptide and an NOI in a sterile tube for about 10 mins at room temperature, resulting in a condensed polypeptide/NOI complex. A common technique is to spot the nucleic acid and protein alongside each other in the tube, but not in contact, and initiate mixing by adding a few hundred microlitres of a liquid carrier, such as a pharmaceutically acceptable carrier, excipient or diluent.

A further and preferred method of preparing a delivery vector/complex of the present invention is by contacting a Mu1 Adenoviral packaging polypeptide and an NOI during continuous vortexing.

Typically a ratio of NOI to polypeptide at least 1:1, preferably from 1:1 to 2:1, more preferably from 1.4:1 to 1.9:1, more preferably from 1.5:1 to 1.8:1, is used. We have found a ratio of NOI to polypeptide of approximately 1:0.6 (~1.7:1) to be particularly effective. In some aspects, typically a ratio of polypeptide to NOI of from 0.2 to 1.5, preferably from 0.3 to 1.2 (w/w), more preferably from 0.5 to 0.7 is used. In other embodiments the typically ratio of polypeptide to NOI is at least 10:1, or at least 20:1 (w/w). However, the optimum ratio may depend on the charge : amino acid ratio of the Mu1 Adenoviral packaging polypeptide. Generally, the lower the charge : amino acid ratio, the higher the polypeptide : NOI ratio used.

Next, cationic lipids are added to the complex. The cationic lipids may, in one embodiment, be part of a pre-formed liposome comprising two or more lipid constituents, such as DC-Chol and DOPE. The cationic lipids are typically incubated with the polypeptide/NOI complex for about 20 mins at room temperature. A further and preferred method of adding the cationic lipids is in the form of a cationic liposome suspension. This final complex may be stored at approximately -80°C with the addition of 10% sucrose (w/v) until use.

The amount of liposome to NOI is typically in the order of from 3:1 to 20:1, preferably from 6:1 to 15:1, more preferably from 8:1 to 14:1. We have found a ratio of liposome to NOI of 12:1 to be particularly effective. In other embodiments the amount of liposome to NOI is typically in the order of the 2:1 to 10:1, or from 3:1 to 6:1. Where cationic lipids are used with neutral lipids, the ratio is typically in the order of 1:1.

In a highly preferred embodiment the ratio

| | liposome : | NOI : | polypeptide |
|---|---|---|---|
| is | 3-20 : | 1 : | 0.5-1 |
| preferably | 8-14 : | 1 : | 0.5-0.7 |
| more preferably | ~12 : | 1 : | ~0.6 |

The delivery vector/complex is now ready for use. Although it is preferred to mix the various components in the order described above, it is possible to combine the components in any order. Where further polypeptide components are to be added, they may be added at any stage but preferably together with the Mu1 Adenoviral packaging polypeptide.

It may be desirable to include other components within the vectors/complexes, for example ligands that bind to cell surface receptors, to provide the vectors/complexes with a degree of selectivity for cell type. Ligands include peptides, glycoproteins, oligosaccharides, lectins and antibodies and fragments thereof.

### E. Administration

The delivery vector/complex of the invention is preferably combined with a pharmaceutically acceptable carrier or diluent to produce a pharmaceutical composition (which may be for human or animal use). Suitable carriers and diluents include isotonic saline solutions, for example phosphate-buffered saline. The composition of the invention may be administered by direct injection. The composition may be formulated for parenteral, intramuscular, intravenous, subcutaneous, intraocular or transdermal administration or inhalation. Typically, each NOI may be administered at a dose of from 10 ng to 10 *µ*g/kg body weight, preferably from 0.1 to 10 *µ*g/kg, more preferably from 0.1 to 1 *µ*g/kg body weight.

Alternatively, transfection of patient cells may be carried out *ex vivo* by removal of patient tissue, transfection using a delivery vector/complex of the invention, followed by reimplantation of the transfected tissue.

The routes of administration and dosages described are intended only as a guide since a skilled practitioner will be able to determine readily the optimum route of administration and dosage for any particular patient and condition.

### F. Uses

The delivery vectors/complexes in the present invention may be used to efficiently transfect eukaryotic cells, in particular mammalian cells, with NOIs. The delivery vectors/complexes have been shown to be particularly efficient compared with prior art compositions in transfecting neuronal cells. This has specific implications for (i) research where neuronal cells are used and (ii) clinical applications where it is desired to introduce NOIs into cells of the central of peripheral nervous system of a human or animal. More generally, the delivery vectors/complexes in the present invention may be used in a variety of NOI delivery applications such as gene therapy, DNA vaccine delivery and *in vitro* transfection studies.

Examples of diseases that may be targeted for treatment using the complexes/vectors of the invention include diseases of the peripheral or central nervous system such as neurodegenerative diseases and damage to nervous tissue as a result of injury/trauma (including strokes). In particular, neurodegenerative diseases include motor neurone disease, several inherited diseases, such as familial dysautonomia and infantile spinal muscular atrophy, and late onset neurodegenerative diseases such as Parkinson's and Alzheimer's diseases.

The delivery vectors/complexes of the invention may also be used to administer therapeutic genes to a patient suffering from a malignancy. Examples of malignancies that may be targeted for treatment include cancer of the breast, cervix, colon, rectum, endometrium, kidney, lung, ovary, pancreas, prostate gland, skin, stomach, bladder, CNS, oesophagus, head-or-neck, liver, testis, thymus or thyroid. Malignancies of blood cells, bone marrow cells, B-lymphocytes, T-lymphocytes, lymphocytic progenitors or myeloid cell progenitors may also be targeted for treatment.

The tumour may be a solid tumour or a non-solid tumour and may be a primary tumour or a disseminated metastatic (secondary) tumour. Non-solid tumours include myeloma; leukaemia (acute or chronic, lymphocytic or myelocytic) such as acute myeloblastic, acute promyelocytic, acute myelomonocytic, acute monocytic, erythroleukaemia; and lymphomas such as Hodgkin's, non-Hodgkin's and Burkitt's. Solid tumours include carcinoma, colon carcinoma, small cell lung carcinoma, non-small cell lung carcinoma, adenocarcinoma, melanoma, basal or squamous cell carcinoma, mesothelioma, adenocarcinoma, neuroblastoma, glioma, astrocytoma, medulloblastoma, retinoblastoma, sarcoma, osteosarcoma, rhabdomyosarcoma, fibrosarcoma, osteogenic sarcoma, hepatoma, and seminoma.

Other diseases of interest include diseases caused by mutations, inherited or somatic, in normal cellular genes, such as cystic fibrosis, thalessemias and the like.

Further areas of interest include the treatment of immune-related disorders such as organ transplant rejection and autoimmune diseases. The spectrum of autoimmune disorders ranges from organ specific diseases (such as thyroiditis, insulitis, multiple sclerosis, iridocyclitis, uveitis, orchitis, hepatitis, Addison's disease, myasthenia gravis) to systemic illnesses such as rheumatoid arthritis, and other rheumatic disorders, or lupus erythematosus. Other disorders include immune hyperreactivity, such as allergic reactions, in particular reaction associated with histamine production, and asthma.

The present invention will now be illustrated by means of the following examples which are illustrative only and not limiting.

### Description of the figures

Figure 1 shows a plate;
Figure 2 shows a graph;
Figure 3 shows a plate;
Figure 4 shows a graph;
Figure 5 shows a graph;
Figure 6 shows a graph;
Figure 7 shows a graph;
Figure 8 shows a graph;
Figure 9 shows structures;
Figure 10 shows a graph;
Figure 11 shows a graph;
Figure 12 shows a graph;
Figure 13 shows a graph;
Figure 14 shows a graph;
Figure 15 shows a graph;
Figure 16 shows a plate;
Figure 17 shows a graph;
Figure 18 shows a graph;
Figure 19 shows a structure;
Figure 20 shows a reaction scheme;
Figure 21 shows a reaction scheme;
Figure 22 shows structures;
Figure 23 shows principle of miscellar incorporation;
Figure 24 shows a graph; and
Figure 25 shows a graph.

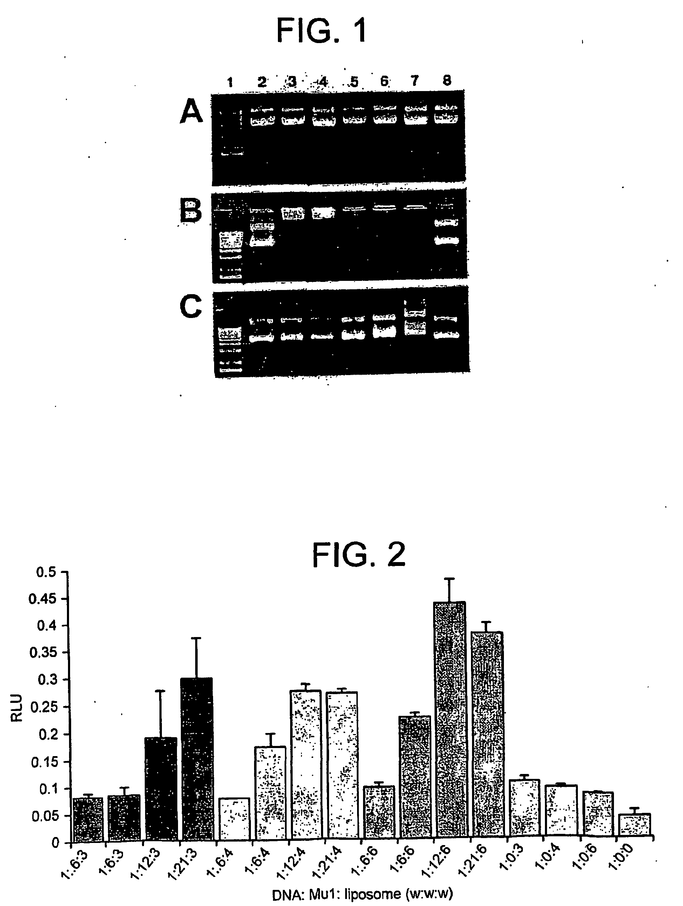

### Detailed description of the figures 1 to 6

### Figure 1- The Adenoviral core protein Mu1 is more efficient at binding plasmid DNA than Polyomavirus core protein Vp1.

A) BSA has no effect on the electrophoretic mobility of pDNA. One microgram of pCMVβ was incubated with 0 µg (lane 2), 5 µg (lane 3), 10 µg (lane 4), 15 µg (lane 5), 20 µg (lane 6), 25 µg (lane 7) and 30 µg (lane 8) of BSA for 10 minutes at room temperature in 1X HBS. Samples were then analyzed on a 1% agarose gel for altered mobility. No change in electrophoretic mobility by BSA was detected.
B) In contrast to BSA, Mu1 peptide dramatically interfered with the mobility of pDNA. pCMVβ (1 µg) was incubated with 0.25 µg (lane 2), 0.5 µg (lane 3), 1 µg (lane 4), 2 µg (lane 4), 4 µg (lane 6), 6 µg (lane 7) and 0 µg (lane 8) recombinant Mu1 peptide as in A. While ratios of protein to pDNA of 0.25 (w/w) (lane 2) did not alter migration of the relaxed form of pCMVβ (upper band) a slight retardation of supercoiled pDNA was seen (lower band). When ratios of 0.5 (w/w) or greater were used, however, migration of both forms of pDNA was severely retarded.
C). The Polyomavirus protein Vp1 was much less efficient at preventing pDNA migration. pCMVβ (1 µg) was incubated with 2 µg (lane 2), 4 µg (lane 3), 6 µg (lane 4), 8 µg (lane5), 16 µg (lane 6), 32 µg (lane 7) and 0 µg (lane 8) Vp1. Only ratios of 6 or higher (protein: pDNA, w/w) caused significant retardation of supercoiled pDNA (lane 6, lower band). Also, not until a ratio of 32 (w/w) was used was there any effect on relaxed pDNA (lane 7, upper band). In all gels lane 1 corresponds to 1 Kb DNA marker (BRL).

### Figure 2 - β Galactosidase activity in ND7 cells transfected with pDNA-Mu1-cationic liposome complexes.

ND7 cells were seeded at a density of 5 x 10⁴ cells/well in 24 well culture dishes 24 hrs prior to transfection. Immediately prior to transfection, cells were washed in serum-free media. Complexes were formed by incubating pCMVβ with Mu1 prior to the addition of the cationic liposome DC-Chol/DOPE. In each case 1 µg pCMVβ was complexed with 0.6, 6, 12, and 21 µg Mu1 peptide. Each of these combinations was then complexed with 3, 4 and 6 µg DC-Chol/DOPE. ND7 cells were exposed to transfection complexes for 2 hours then maintained at 37°C, 5 % CO₂ for another 24 hrs before being harvested and processed for β-galactosidase enzyme assay. Numbers represent means ± SD, n=3.

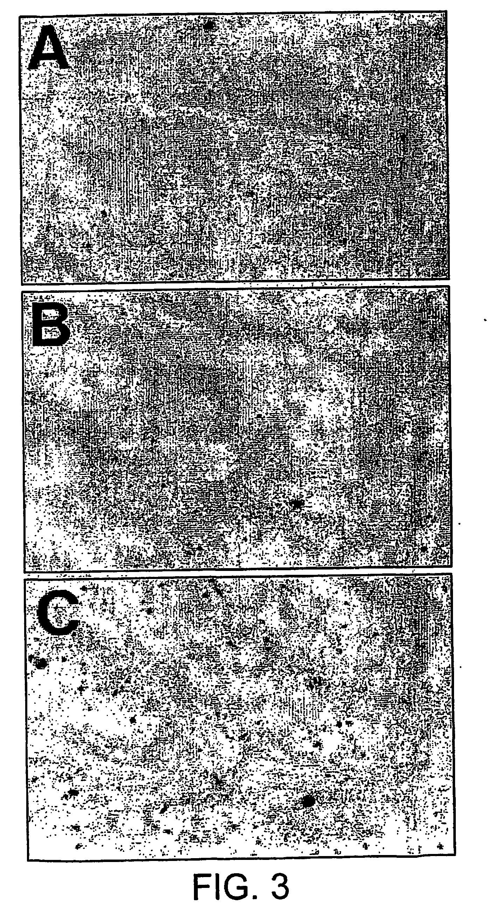

### Figure 3 - Mu1 enhances cationic liposome mediated transfection efficiency in the neuronal cell line ND7.

ND7 neurons were plated in 24 well culture dishes at a density of 4X10⁴ cells/well and allowed to grow for 24 hrs. The undifferentiated ND7 neurons were then transfected with either pCMVb alone (A), pCMVb complexed with DC-Chol/DOPE (1/3, w/w) (B) or with pCMVb complexed with Mu1 and DC-Chol/DOPE (1/12/6) (C). Forty-eight hours later the cells were fixed and processed for histochemical detection of X-Gal. As can be seen in panel C inclusion of Mu1 in the complex at an optimal ratio significantly enhanced the number of X-Gal positive cells (blue).

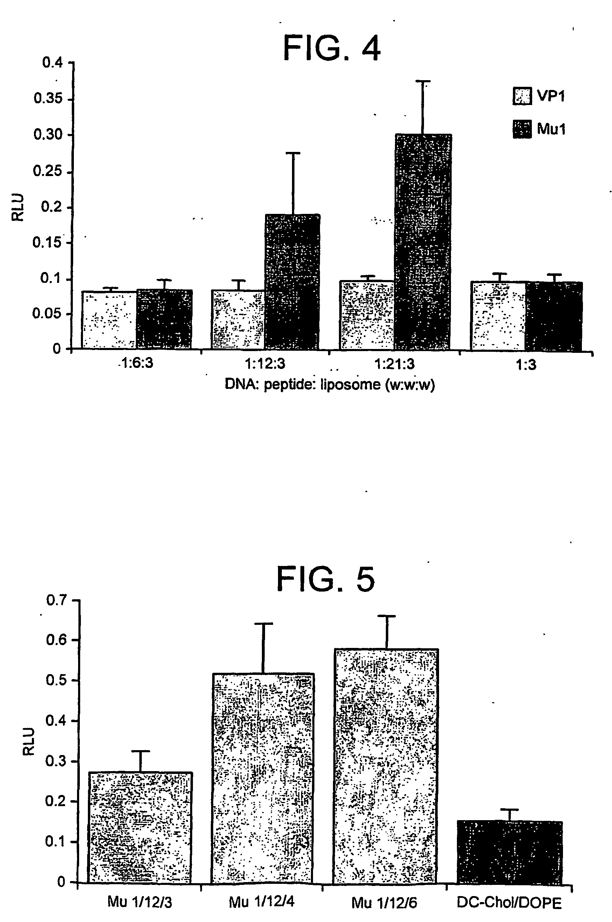

### Figure 4 - Mu1 is more efficient at enhancing cationic liposome mediated transfections in ND7 cells than Vp1

pCMVβ plasmid DNA was complexed to various amounts of polycationic peptide and then mixed with cationic liposome at a ratio of 1:3 (pCMVβ: liposome; w/w). After being washed briefly in serum free media, ND7 cells were exposed to the liposome-polycation-liposome complexes for two hours and then returned to serum containing media. Twenty-four hours later the cells were harvested and processed for β-galactosidase enzyme assay. Each condition was performed in triplicate and each experiment replicated three times. Numbers represent means ± SD.

### Figure 5 - Mu1 enhances DC-Chol/DOPE transfection in COS-7 cells.

COS cells were seeded at a density of 60-80% confluence in 24 well culture dishes 24 hrs prior to transfection. Immediately prior to transfection, cells were washed in serum-free media. Incubating pCMVβ with Mu1 prior to the addition of the cationic liposome DC-Chol/DOPE formed complexes capable of cellular transfection. In each case 1 µg pCMVβ was complexed with 12 µg Mulpeptide that had been found optimal for ND7 cells. The pCMVβ: Mu1 complexes were then mixed with 3, 4 and 6 µg DC-Chol/DOPE. COS cells were exposed to transfection complexes for 2 hours then maintained at 37°C, 5% CO₂ for another 24 hrs before being harvested and processed for β-galactosidase enzyme assay. Numbers represent means ± SD, n=3.

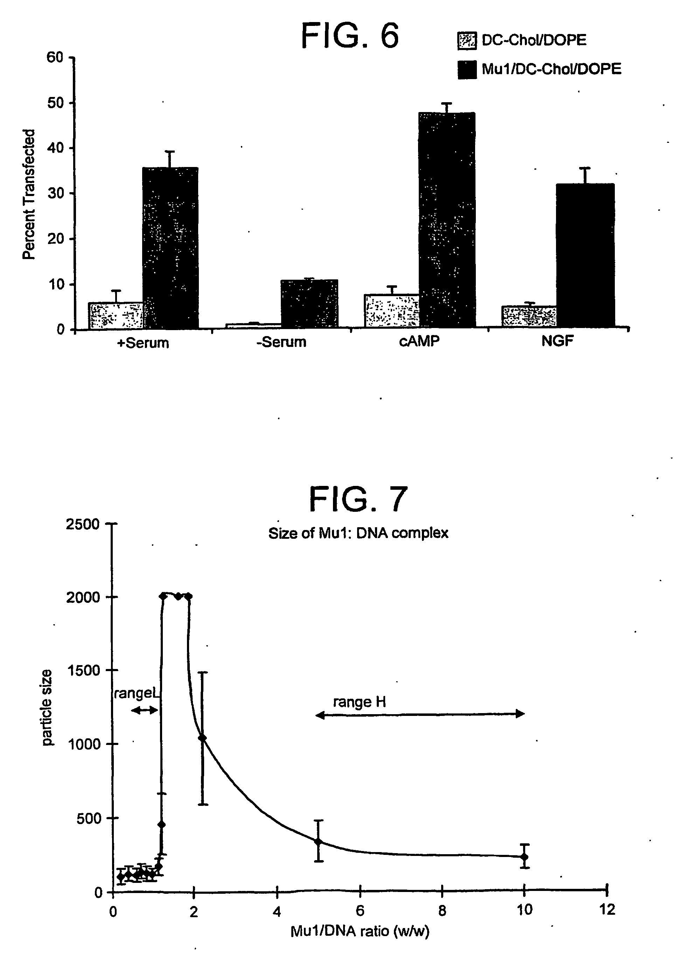

### Figure 6 - Transfection efficiency in differentiated ND7 cells with pCMVβ-Mu1-cationic liposome complexes.

ND7 cells were plated in a 24 well culture plate at a density of 4X10⁴ cells per well in normal growth media (+ serum). Twenty-four hours later the media was replaced with differentiation media and the cells were grown for an additional 24 hrs. Three different differentiation medias were used; serum-free (-serum), normal growth media plus 1 mM cAMP (cAMP), or reduced serum (0.5 %) plus 1 mM cAMP and 50 ng/ml nerve growth factor (NGF). The cells were then transfected with pCMVb complexed with either DC-Chol/DOPE alone or Mu1 plus DC-Chol/DOPE. Forty-eight hours later the cells were fixed and processed for X-Gal histochemistry and the percentage of positive cells determined. In all cases the presence of Mu1 increased the number of positive cells.

Interestingly the number of cells transfected was greater both with and without Mu1 for cells grown in cAMP.

### EXAMPLES

### Materials and Methods

### Peptide Synthesis

Peptides Vp1 and Mu1 were synthesized on a Shimadzu PSSM-8 solid phase peptide synthesizer using a five-fold excess of (9-fluorenyl)methoxycarbonyl (Fmoc)-protected L-amino acids (Novabiochem) and the FastMoc™ reagents 2-(1H-benzotriazole-1-yl)-1,1,3,3-tetra-methyluronium hexafluorophosphate/hydroxybenzotriazole (HBTU/HOBt) (Advanced Chemtech Europe) as the amide coupling agent. After resin cleavage and deprotection, desalting was performed by gel filtration using a column of P2 Biogel (2 x 28 cm; Biorad) attached to an FPLC system (Amersham Pharmacia Biotech UK) with 0.1% aqueous TFA as eluant at a flow rate of 0.5-0.75 ml/min. Final preparative reverse-phase purification was achieved with a Vydac column (C18, 5 µm, 2 x 25 cm; Hichrom) attached to a Gilson HPLC system (Anachem). Peptides were eluted at 5ml/min by means of a linear gradient of acetonitrile in 0.1% aqueous TFA and elution monitored at 220-230 nm.

The Vp1 peptide was prepared using a preloaded *L*-Pro-2-chlorotrityl super acid labile resin (Novabiochem) (100 mg, 1.05 mmol/g, 0.1 mmol). Extended coupling times were used to incorporate all amino acid residues from the sixth (Lys) through to the *N*-terminal residue. After automated *N*-terminal Fmoc deprotection with piperidine (20%, v/v) in dimethyl formamide, the resin was isolated, washed with dimethylformamide (10 ml) and methanol (15 ml), and then dried *in vacuo.* Crude peptide was cleaved from the resin using ice cooled TFA (8 ml), containing phenol (7%, w/v), ethanedithiol (2%, v/v), thioanisole (4%, v/v) and water (4%, v/v) (known as Mixture A), and then precipitated with ice cold methyl-*tert*-butylether (MTBE) (30ml). The subsequent pellet was then desalted and the crude peptide mixture purified by reverse phase HPLC. After elution, fractions containing the desired peptide (eluting with acetonitrile 68.5% v/v) were combined and lyophilized to give the peptide as a white powder. Overall yield: 32 mg (15 µmol, 15%); MS (MALDI-TOF) C₈₅H₁₅₁N₂₆O₂₆S₃: [*M* + H]⁺ calcd 2049.5, found 2050.2. The sequence was confirmed by amino acid composition and sequence analysis. Homogeneity was judged >95% by HPLC analysis.

The Mu1 peptide was prepared using Gly-Wang resin (Novabiochem) (40 mg, 0.67 mmol/g, 0.03mmol). Normal coupling times were used throughout. After automated *N-*terminal Fmoc deprotection as above, the resin was isolated and washed with dichloromethane (20 ml) and methanol (20 ml) after which the resin was dried *in vacuo.* Crude peptide was cleaved from the resin using Mixture A (8 ml) and precipitated with MTBE (30 ml), all as above. Finally, the crude peptide mixture was desalted and purified by reverse phase HPLC. After elution, fractions containing the desired peptide (eluting with acetonitrile 17.2%) were combined and lyophilized to give the peptide as a white powder. Overall yield: 65 mg (26 µmol, 80%); MS (ES) C₉₅H₁₇₀N₅₂O₂₁ S₂: [*M* + H]⁺ calcd 2440.7, found 2440.6. Homogeneity was judged >95% by HPLC analysis.

### DNA binding analysis

The purified peptides were reconstituted in sterile distilled H₂O at 3 mg/mL. Peptide and pDNA were complexed in 20 µL HEPES buffered saline (137 mM NaCl, 5 mM KCl, 0.75 mM Na₂HPO₄, 19 mM HEPES, pH 7.4) for 20 minutes at room temperature. Peptide: pDNA complexes were subsequently analyzed by agarose gel electrophoresis (1%). Control incubations for general macromolecular pDNA interactions were performed with varying amounts of molecular biology grade purified bovine serum albumin (Sigma).

### Cell Cultures

ND7s are a well-characterized cell line derived from the fusion of a neuroblastoma (N18Tg2) with neonatal rat sensory neurons ²⁸. The cell line was maintained in normal growth media (NGM) (Leibovitz's L-15 media (BRL) enriched with 10 % Fetal bovine serum (BRL), 4 g/L glucose, 4 g/L sodium bicarbonate (BRL), 100 IU/mL penicillin/streptomycin (BRL)) at 37°C and 5 % CO₂. The cells were plated onto 24 well plates (Costar) at a density that produced 70 % confluence after 24 hours.

Differentiation of ND7 cells was carried out using three previously described methods ²⁸, ²⁹_{.} ND7 cells were seeded in NGM at a density of 4 X 10⁴ cells per well in a 24 well culture dish (Nunc). Twenty four hours later the media was replaced with either: a) NGM supplemented with 1mM adenosine 3':5'-cyclic monophosphate (cAMP; Sigma), or b) serum-free differentiation media (50 % Hams F12, 50 % DMEM, 5 µg/mL Transterrin, 250 ng/mL Insulin, 0.3 µM sodium selenite), or c) low serum nerve growth factor (NGF) media (L-15 supplemented with 2 mM glutamine, 4 g/L glucose, 4 g/L sodium bicarbonate, 10 u/mL penicillin, 10 g/mL streptomycin, 0.5% FCS, 1 mM cAMP, 50 ng/mL NGF (Alomone Labs)). Differentiated ND7 cells were grown in appropriate media for 24 hrs at 37°C, 5 % CO₂ prior to transfection.

COS-7 cells (derived from Green Monkey kidney) were grown in RPMI 1640 media (BRL) supplemented with 10% fetal bovine serum (BRL) and 100 IU/mL penicillin/streptomycin (BRL).

### Plasmid Constructs

All transfections utilized the reporter plasmid pCMVβ (Clontech, Palo Alto, CA) containing the full-length sequence for *E. coli* β-galactosidase downstream of the human cytomegalovirus immediate-early promoter/enhancer (Clontech). Stocks of plasmid DNA were prepared using standard molecular cloning techniques and purified using the Qiagen Endotoxin-free plasmid purification system (Qiagen, Dorking, UK).

### Liposomes

DC-Chol/DOPE liposomes were prepared as previously described ^{30, 31}. Briefly, 6 µmol of DC-Chol and 4 µmol of DOPE (supplied at 10mg/mL in CHCl₃) were added to freshly distilled CH₂Cl₂ (5 mL) under nitrogen. 5 mL of 20 mM Hepes (pH 7.8) was added to the mixture and this was sonicated for 3 minutes. The organic solvents were removed under reduced pressure and the resulting liposome suspension was then sonicated for a further 3 minutes. Liposome preparations were stored at 4°C.

### Transfection Protocol

Since initial experiments determined that the presence of fetal bovine serum inhibited transfection of ND7 cells serum-free differentiation media was used for all transfections. Various amounts of DNA and liposome were placed in the bottom of a 7 mL sterile Bijou container (Bibby Sterilin Ltd., Staffordshire, U.K.), but not in contact with each other.

DNA and liposomes were combined by the addition of 400 µL serum-free differentiation media and gentle shaking. The DNA: liposome mixture was incubated at room temperature for 20 to 30 minutes before being applied to the cells. The DNA/liposome mixture was then applied to the cells and incubated at 37°C, 5% CO₂ for 2 hours after which this media was replaced with complete media. Twenty four to 48 hours later the cells were fixed and processed for X-gal histochemistry as described ³¹ or harvested for β-galactosidase enzyme assays (Promega Corp.).

Cell counts were performed under x40 magnification using a Nikon Diaphot inverted microscope. Each transfection was repeated at least three times and at least three separate counts were made for each well.

Transfection complexes including the test peptides were generated in the following manner. Various amounts of peptide was placed in the bottom of sterile polystyrene containers alongside, but not in contact with 1 µg pCMVβ and mixed by adding 400 µl serum free NGM media. The complexes were incubated at room temperature for 10 minutes after which DC-Chol/DOPE was added. The pDNA/peptide/liposome complex was further incubated at room temperature for 20 minutes and then administered to cells as above.

### Example 1- Comparing the DNA binding capacity of Mu1 and Vp1

Mu1 is a polycationic peptide comprised of 19 amino acids associated with the core complex of Adenovirus (Table 1) ^{27, 32}. We compared the DNA binding capacity of Mu1 with the mouse polyomavirus major capsid protein Vp1 by interaction with plasmid DNA in a gel retardation assay. Vp1 is a 19 amino acid peptide that contains a nuclear localization signal ²⁶ and contains fewer positively charged amino acids than Mu1. It was therefore predicted to have a lower DNA binding capacity.

**Table 1 Mu1 and VP1 protein sequences**

| Polypeptide | Sequence | MW | Charge/AA ratio |
|---|---|---|---|
| Mu1 | NH₂-Met-Arg-Arg-Ala-His-His-Arg-Arg-Arg-Arg-Ala-Ser-His-Arg-Arg-Met-Arg-Gly-Gly-OH | 2440 | 0.63 |
| VP1 | NH₂-Met-Ala-Pro-Lys-Arg-Lys-Ser-Gly-Val-Ser-Lys-Cys-Glu-Thr-Lys-Cys-Thr-Pro-Pro-OH | 2049 | 0.26 |

The NLS sequence in VP1 is underlined

Varying amounts of purified peptide were incubated at room temperature in HBS for approximately 10 minutes and then analyzed by agarose gel electrophoresis. Without the addition of peptide, supercoiled and relaxed circular plasmid DNA (pDNA) migrated in the expected manner (Figure 1, lane 8).

Beginning at a DNA: Mu1 peptide ratio of 1:0.25 (w/w) the migration of plasmid DNA was retarded (Figure 1). The migration of plasmid DNA was slightly affected at 1:0.25 ratios (w/w), but at a ratio 1:0.5 (w/w) the migration of plasmid DNA was severely slowed and very little managed to migrate out of the wells. At ratios of 2:1 and above pDNA was unable to migrate into the agarose gel and the ability of ethidium bromide to interchelate into the plasmid was reduced. In contrast to Mu1, no effect on the electrophoretic mobility of plasmid DNA was detected with Vp1 at pDNA: protein ratios up to 1:8 (w/w) (Figure 1). The addition of 8 µg Vp1 to 1 µg plasmid DNA resulted in a broadening of the supercoiled pDNA band. However, no effect was seen on the relaxed pDNA band with Vp1 until a ratio of 1:32 (w/w) pDNA: protein was used. At this ratio, both supercoiled and relaxed pDNA bands were significantly retarded and some DNA could be seen retained in the well. No effect on electrophoretic mobility was detected when pDNA was incubated with BSA at ratios up to 1:30 (w/w) pDNA: protein (Figure 1).

### Example 2 - Comparing the ability of Mu1 and Vp1 to enhance transfection in undifferentiated ND7s

We examined the ability of Mu1 and Vp1 to enhance the transfection of a neuronal cell line by cationic liposomes using a β-Galactosidase reporter gene assay. ND7 cells were transfected with pCMVβ complexed to varying amounts of peptide and DC-Chol/DOPE. We have previously shown that the cationic liposome DC-Chol/DOPE is capable of efficiently transfecting the neuronally derived ND7 cell line ³¹. In this study we found that optimal efficiencies (>40%) were obtained in this neuronally derived cell line using 1 µg plasmid DNA complexed with 3 µg DC-Chol/DOPE ³¹. Temporally, maximal levels of transgene expression are obtained between 48-60 hours post transfection. Therefore, in order to maximize the chance of detecting improvements in transfection we performed all our assays within 12-20 hours of transfection at a time when levels of reporter gene expression were lower. Previously we found a pDNA: liposome ratio of 1:3 (w/w) optimal for transfections in ND7 cells ³¹. We therefore compared the effect of various amounts of peptide on transfections at ratios of 1:3, 1:4 and 1:6 pDNA: DC-Chol/DOPE.. The gel retardation analysis suggested that an approximate ratio of 1:0.5 (w/w), pDNA: Mu1 was enough to essentially bind all of the plasmid DNA (Figure 1). However, initial experiments using this ratio and liposomes did not affect transfection efficiencies (not shown). The volumes used to generate transfection complexes were much larger than those used to perform the gel retardation assay (400 µL vs. 20 µL). Therefore we tested larger quantities of Mu1 that were of a similar concentration in solution as that used in the gel retardation assay. We compared the effect 0.6, 6, 12 and 21 µg of Mu1 peptide would have on DC-Chol/DOPE mediated transfections. We found that Mu1 was able to improve cationic liposome mediated transfection efficiencies over 4-fold. The greatest improvement in transfection efficiencies occurred when the relative ratios of 1/12/6, pCMVβ/Mu1/(DC-Cho1/DOPE) (w/w/w) were used. This combination led to an 11-fold increase in transfections compared to DNA alone (Figure 2).

The β-galactosidase reporter gene assay provides a measure of the overall level of β-galactosidase produced, but gives no information regarding the number of cells transfected. For this reason, we also performed cell counts on transfected ND7 cells. Cells were seeded at a density of 4 x 10⁴ in 24 well culture plates. After 24 hours the cells were washed briefly in serum-free media and transfected with pCMVβ complexed to DC-Chol/DOPE and Mu1 peptide. The ratios used were those found to be optimal in the reporter gene assay, 1:12:6, pCMVβ:Mu1:DC-Chol/DOPE. Using these ratios, we found a 6-fold increase in the number of β galactosidase positive cells (Figures 3 & 6). No obvious cell loss was detected with the Mu1 complex at any of the concentrations utilized. Similarly the concentration of protein in the cellular lysates used for β-Galactosidase reporter gene assay did not significantly vary with untransfected cells (data not shown).

In contrast, no improvement on transfection efficiency could be found with Vp1 (Figure 4). No improvement in transfection efficiencies over naked DNA was seen with pCMVβ complexed to Mu1 alone.

In order to see whether improved transfections could be achieved in other cell types we performed a similar analysis on COS-7 cells. Mu1 also improved liposome-mediated transfection in COS-7 cells (Figure 5). The same ratio of pDNA: Mu1 : liposome optimal for ND7 cells was best for COS-7 cells. A similar degree of improvement was also seen (3.7 fold) over cationic liposomes alone.

### Example 3 - Transfection in differentiated ND7s

We also examined the ability of Mu1 to improved cationic liposome-mediated transfection in differentiated ND7s. The ND7 cell line is derived from a fusion of primary rat dorsal root ganglia (DRG) neurons and the mouse neuroblastoma N18Tg2 ²⁸. ND7 cells can be differentiated in a variety of manners including the withdrawal of serum, cAMP administration or exposure to reduced serum plus cAMP and nerve growth factor. Differentiation of ND7s leads to the expression of cellular properties associated with their parental nociceptive sensory neurons including a reduction in cell division and the onset of neurite outgrowth. ND7 cells were seeded in 24 well culture plates and 24 hours later differentiated. Fifteen to 20 hours following the onset of differentiation, they were transfected as above. Fifteen to 20 hours following transfection, cells were fixed and processed for X-gal histochemistry. Consistent with previous observations, transfection efficiencies varied greatly between the three differentiated groups. ND7 cells differentiated by withdrawal of serum exhibited the lowest levels of transfection (1.3%) while highest levels were seen in the cAMP group (8%) and intermediate levels in the low serum/cAMP/NGF group (4.7%) (Figure 5). In all three conditions, however, inclusion of Mu1 polypeptide in the transfection complex improved the transduction of differentiated ND7 cells. ND7s differentiated by either cAMP alone or exposure to low serum/cAMP/NGF exhibited increased efficiencies of greater than 6 fold (Figure 5). The greatest improvement in efficiencies was seen, however, in the group differentiated by serum withdrawal. Here, increases of greater than 10 fold were observed.

### Complexes of Mu1 peptide and DNA

As shown in Example 1 (DNA binding Analysis) using gel electrophoresis, the migration of plasmid DNA was severely retarded and little DNA migrated out of the wells above a Mu1:DNA 0.5:1.0 (w/w). This implied that Mu1 peptide was strongly interacting with DNA and might neutralise and condense nucleic acids to form small particles suitable for gene delivery. The size of Mu1:DNA (MD) particle sizes were examined over the Mu1: DNA ratio range indicated in Fig.7.

MD particles were prepared by mixing. Briefly, appropriate aliquots of Mu1 peptide in deionized water were added to plasmid DNA (pCMVβ) (final concentration 220µg/ml) in 20 mM Hepes buffer, pH7.0. After mixing well, each mixture was incubated for 10 min at 20°C. Immediately after incubation each mixture was diluted with the Hepes buffer (final DNA concentration 24µg/ml) and subjected to particle size analysis by photon correlation spectroscopy (N4 plus, Coulter). All measurements were performed at 20°C and data collected at an angle of 90°. Unimordal analysis was used to calculate the mean particle size and standard distribution (S.D.).

Interestingly, though Mu1 bound DNA and formed complexes over the complete range examined, the particle size varied considerably in response to the Mu1:DNA ratio. Stable, small nano-particles were formed within the Mu1:DNA ratio 0.3 to 1.2 ( range L) and over 5 (range H). Intermediate ratios resulted in heavy aggregation with the size of complex particles growing over the time of incubation to reach more than 2µm in size (Fig .7).

### Example 4 - Preparation of LMD in the range L

We determined whether liposome-Mu1-DNA complexes with a low MD:DNA ratio could form stable nano-particles and whether the resulting complex particles could have good transfection activities.

Preparation of liposomes; DC-Chol (30µmol) and DOPE (20µmol) were combined in dichloromethane. The organic solvent was removed under reduced pressure using a rotary evaporator and the residue dried for 3h *in vacuo.* Following this, 4mM Hepes buffer, pH7.0 (3ml), was added to the lipid film with vortex mixing. After brief sonication (2-3min), the resulting cationic liposome suspension was extruded by means of an Extruder device (Lipex Biomembranes) three times through two stacked polycarbonate filters (0.2µm Millipore) and then ten times through two stacked polycarbonate filters (0.1µm Millipore) to form small liposomes (109nm average diameter by PCS) (approx. 8-10 mg/ml depending upon the preparation).

Preparation of Liposome:Mu1:DNA (LMD) complexes; Mu1 peptide (0.12mg in deionised water, peptide concentration 3.5mg/ml) was added to a solution of plasmid DNA (pCF1-CAT) (0.2mg, plasmid concentration typically 1.0mg/ml) in 4mM Hepes buffer during continuous vortexing. Cationic liposome suspension (total lipid 2.4mg, 4 µmol) was then introduced resulting in the formation of small particles with narrow size distribution (168nm±58nm) as measured by PCS. This LMD (final DNA concentration 0.14mg/ml) was stored in -80°C with the addition of 10% sucrose (w/v) until use. No particle size deviation was observed over one month.

Liposome:DNA (LD) complexes (lipoplexes) were prepared for control experiments with a Liposome:DNA ratio of 3:1 (w/w), the optimal composition for transfection ofND7 cells.

Transfection in ND7 cells; ND7 cells were seeded in normal growth medium (NGM) (with 10% serum) at a density of approximately 4x10⁴ cells per well, in a 24-well culture plate. After 24h, cells were washed by brief exposure to NGM (serum free) and then treated with solutions containing LMD or LD complexes, prediluted with NGM (serum free) (final DNA concentration 3.2µg/ml in all cases), for the time periods indicated. Cells were then washed again and incubated for a further 48h prior to harvesting. Levels of transfection were determined by chloramphenicol transferase (CAT) enzyme assay using ¹⁴C-CAM as substrate (Promega). Transfection activity was expressed as a percentage (%) conversion of the imputed ¹⁴C-CAM by the enzyme.

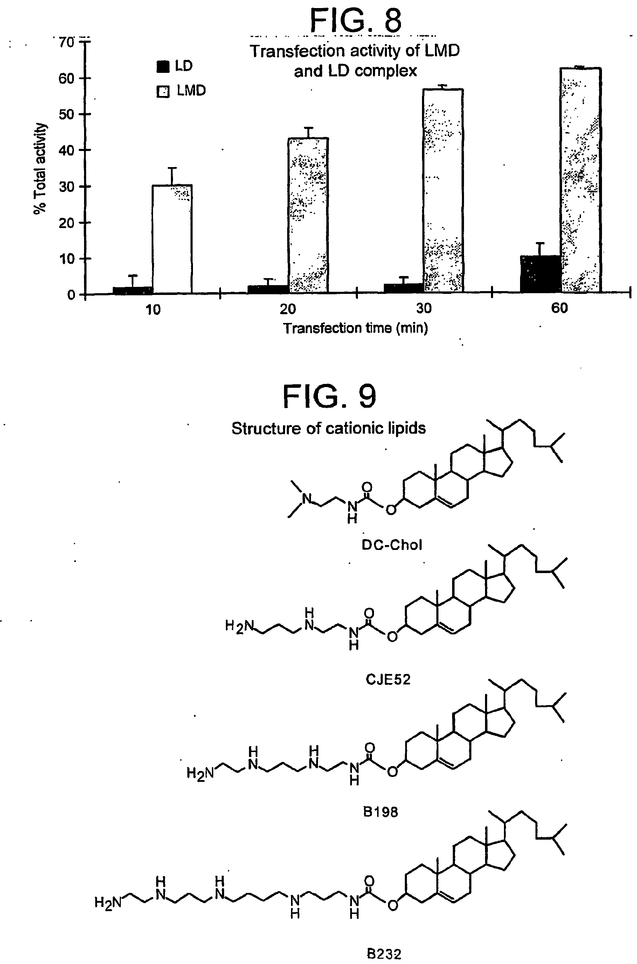

We found much higher reporter gene expression with LMD compared to LD mediated transfection. In fact, LMD transfection resulted in 16 times more CAT enzyme activity after a transfection time of 10 mins, and 6 times as much after a transfection time of 60 min compared with LD-mediated transfection (Fig.8). Significant transfection was observed with LMD even when the transfection time was as short as 10 min. This data illustrates how rapidly LMD particles are able to enter cells.

### Example 5 - Cationic lipid (cytofectin) variations

We determined whether LMD complexes could be bettered by incorporating poly cationic cholesterol lipids (WO 97/45442). CDAN (B198), ACHx (CJE52) and CTAP (B232) (Fig. 9) were used to make cationic liposomes in place of DC-Chol. Each cationic liposome system used was composed of 60mol% of cationic lipid and 40mol% of DOPE and prepared as described in Example 4. The following different LMD complex systems were prepared and compared: LMD(DC-Chol), LMD(B198), LMD(CJE52), and LMD(B232). All LMD systems were prepared with cationic liposomes (total lipid 20µmol) and 0.6mg of Mu1 peptide per 1.0mg of DNA (pCMVβ), as described above. Particles were shown to be under 200nm in diameter.

Liposome:DNA (LD) complex mixtures (lipoplexes) were prepared for control experiments with a Liposome:DNA ratio of 3:1 (w/w), the optimal composition for transfection of ND7 cells.

Transfection in ND7 cells; ND7 cells were seeded in NGM (with 10% serum) at a density of approximately 4x10⁴ cells per well, in a 24-well culture plate. After 24h, cells were washed by brief exposure to NGM (serum free) and then treated with solutions containing LMD or LD complexes, prediluted with NGM (serum free) (final DNA concentration 2.5µg/ml in all cases), for 1h. Cells were then washed again and incubated for a further 48h prior to processing for histochemical staining with X-gal. The number of cells stained blue were counted under an inverted microscope.

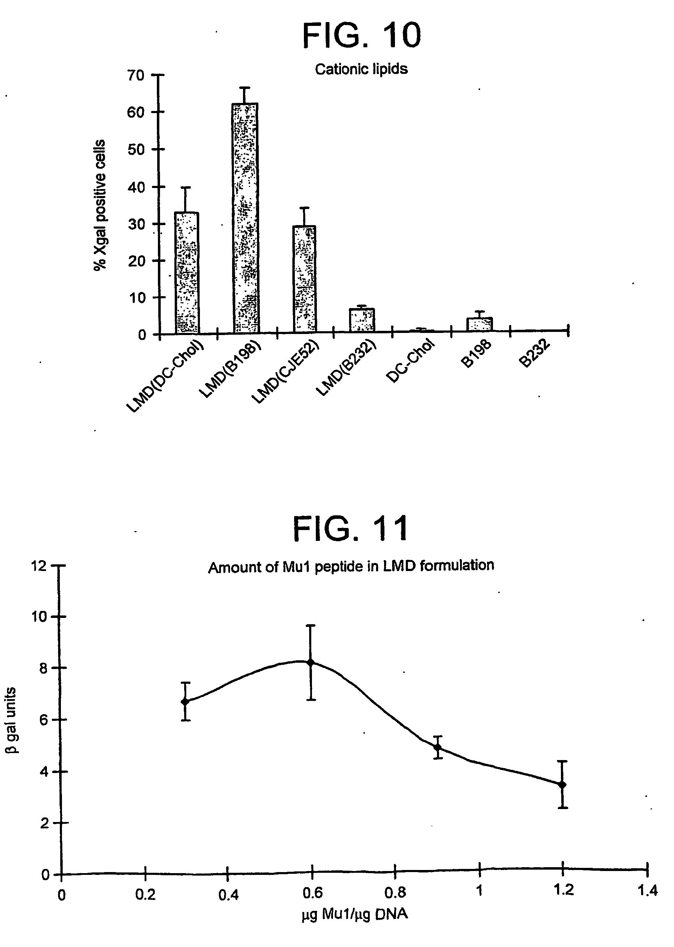

In all cases LMD formulations worked better than corresponding LD systems prepared with the same poly cationic cholesterol lipids (Fig. 10). The rank order in transfection efficiency was LMD(B198) > LMD(DC-Chol) > LMD(CJE52) >> LMD(B232). The same rank order, B198 > DC-Chol > B232, was observed with corresponding LD systems.

### Example 6 - Amount of Mu1 peptide in the range L

We examined the effect of Mu1: DNA ratio (at the range L in Fig.7) on transfection activity.

Cationic liposomes composed of cationic lipid B198 and DOPE (3:2 m/m) were prepared as the same manner described in Example 4. A series of MD complex mixtures (Mu1 :DNA ratio varying from 0.3 to 1.2) were prepared and complexed with the cationic liposome. The resulting LMD systems were comprised of liposome:Mu1:DNA (pCMVβ) in ratios of 12:0.3:1, 12:0.6:0.6, 12:0.9:1 and 12:1.2:1 w/w/w respectively. Measured sizes of LMD particles were approximately 150nm.

Transfection activities were evaluated in vitro using Pane-1 cells (human pancreatic cancer cell line). The cells were seeded at an approximate density of 5x10⁴ per well in a 24-well culture plate in RPMI supplemented with 10% FCS and grown for 24h in the presence of 5% CO₂ at 37°C. Cells were washed by brief exposure to RPMI and then treated with solutions of LMD complexes, prediluted with RPMI (final DNA concentration 5.0µg/ml in all cases), for 30min. Cells were then washed again and incubated for a further 48h in RPMI supplemented with 10% FCS prior to harvesting and the assay of β-galactosidase enzyme activity using a standard assay kit (Promega).

As shown in Fig. 11, the optimum liposome:Mu1 :DNA ratio for transfection of Panc-1 cells was found to be 12:0.6:0.6. Otherwise, excellent transfection results were obtained with these low ratio Mu1 LMD complexes.

### Example 7 - Amount and composition of lipids

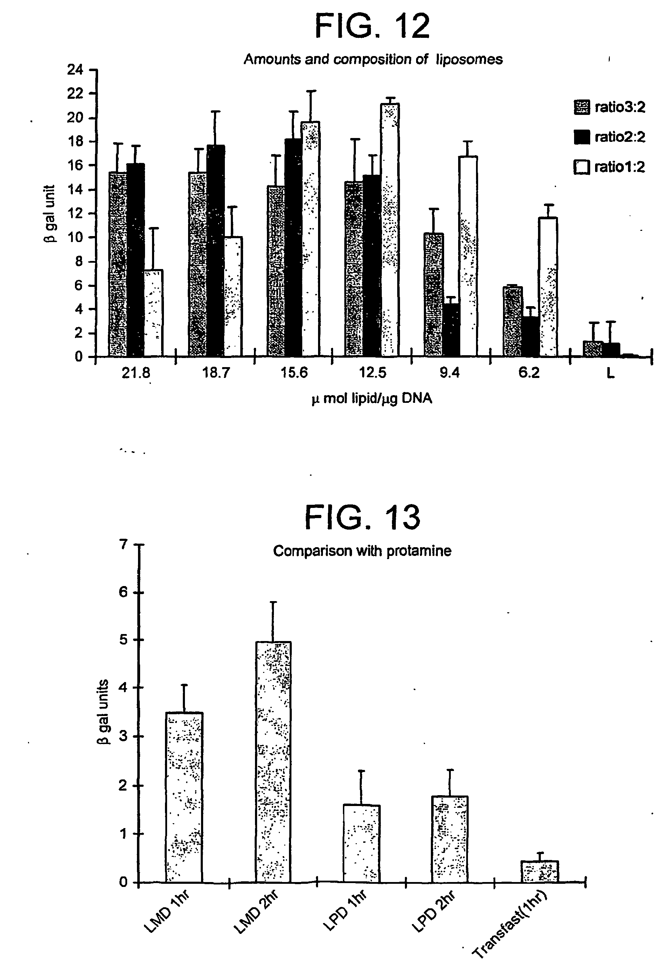

To investigate the effect of varying the ratio of cationic lipid to DOPE as well as the ratio of total lipid to Mu1 and DNA, a series of LMD systems were prepared using B198 as the preferred cationic lipid. Cationic liposomes composed of 60mol% of B198 and 40mol% of DOPE (3:2 m/m), 50mol% of B198 and 50mol% of DOPE (1:1 m/m) and 33mol% of B198 and 67mol% of DOPE (1:2 m/m) were prepared and combined with a standard MD complex mixture (Mul:DNA 0.6:1 w/w) at ratios indicated in Fig.12, according to the method in Example 4.

Liposome:DNA (LD) complex mixtures (lipoplexes) were prepared for control experiments with a Liposome:DNA ratio of 3:1 (w/w). All LMD systems were found to have a larger average size when lower amounts of cationic liposomes were complexed with MD complexes. However, the size of LMD particles composed of more than 12µmol lipids/mg DNA remained less than 200nm, whilst that of 12 to 6µmol lipids/mg DNA climbed above that value. Occasionally, visible aggregation was observed during the preparation of LMD systems comprised of 6µmol lipids/mg DNA.

Transfection activities were determined with Panc-1 cells (Fig.12). The cells were seeded at an approximate density of 5x10⁴ per well in a 24-well culture plate in DMEM supplemented with 10% FCS and grown for 24h in the presence of 5% CO₂ at 37°C. Cells were washed by brief exposure to DMEM and then treated with solutions containing LMD or LD complexes, prediluted with DMEM (final DNA concentration 5.0µg/ml in all cases), for 2h. Cells were then washed again and incubated for a further 48h in DMEM supplemented with 10% FCS prior to harvesting and assay of β-galactosidase enzyme activity using a standard assay kit (Promega).

The maximum transfection activity was not significantly different with the three cationic different lipid to DOPE formulations tested. In the case of LMD systems prepared with B198:DOPE(1:2 m/m) the maximum transfection was achieved at a liposome:DNA ratio of around 12.5µmol lipid /mg DNA. The transfection activities of LMD systems prepared with B198:DOPE (3:2 m/m) and B198:DOPE (2:2 m/m) cationic liposomes reached a plateau at liposome:DNA ratios greater than 12.5µmol lipid/mg DNA. All LMD systems analysed tended to show low transfection activities at low liposome:DNA ratios (Fig. 12). It is considered that LMD formulations composed of low amounts of Mu1 peptide should need larger amounts of cationic liposomes compared to the formulations prepared with higher amounts of Mu1 peptide in order for the respective LMD systems to show full transfection activity.

### Example 8 - Comparison of Mu1 peptide with protamine

Protamine is a naturally occurring cationic peptide abundant in piscine sperm and is potent in neutralising and condensing DNA. The transfection activity of protamine was compared with that of Mu1 peptide. Mu1 peptide or protamine sulfate (Sigma, grade X from Salmon) was complexed with DNA (pCMVβ) and then cationic liposomes (B198:DOPE in a ratio of 3:2 m/m) giving a liposome:peptide:DNA ratio of 12:0.6:1 (w/w/w).

The transfection activities were examined in Swiss 3T3 cells. The cells were seeded at an approximate density of 2x10⁴ per well in a 24-well culture plate in DMEM supplemented with 10% FCS and grown for 48h to complete confluence in the presence of 5% CO₂ at 37°C. Cells were washed by brief exposure to DMEM and then treated with solutions containing LMD or LD complexes, prediluted with DMEM (final DNA concentration 5.0µg/ml in all cases), for 1 or 2h. Cells were then washed again and incubated for a further 48h in DMEM supplemented with 10% FCS prior to harvesting. The level of β-galactosidase enzyme activity was determined with a standard assay kit (Promega).

As shown in Fig. 13 the complexes comprising Mu1 peptide showed better transfection of these confluent cells than those comprising protamine.

### Example 9 - Alternative cationic peptides

In order to examine the effects of various alternative cationic peptides on transfection activities, a series of liposome:cationic peptide:DNA complexes were prepared and their relative transfection abilities analysed *in vitro.* The peptides used were poly-lysine hydrochloride (average molecular weight 3970, Sigma), poly arginine hydrochloride (average molecular weight 11800, Sigma) a peptide derived from protein V, pV (p5, sequence shown below), a peptide analogue of Mu1 (V, sequence shown below) and Mu1 peptide itself. The p5 peptide and V peptide were synthesized using the same solid-phase peptide synthesis methodology as was used to prepare Mu1 peptide.

Each peptide was combined with cationic liposome (DC-Chol:DOPE 3:2 m/m) and DNA (pCMVβ) in the liposome:peptide:DNA ratio of 12:0.6:1 (w/w/w) as described in Example 4. The transfection activities were examined using HeLa cells (human epithelial cells). The cells were seeded at an approximate density of 5x10⁴ per well in a 24-well culture plate in DMEM supplemented with 10% FCS and grown for 24h in the presence of 5% CO₂ at 37°C. Cells were washed by brief exposure to DMEM and then treated with solutions containing LMD or LD complexes, prediluted with OPTIMEM (Gibco) (final DNA concentration 1.0µg/ml in all cases), for 30min. Cells were then washed again and incubated for a further 48h in DMEM supplemented with 10% FCS prior to harvesting. The level of β-galactosidase enzyme activity was determined with a standard assay kit (chemiluminescent, Roche).

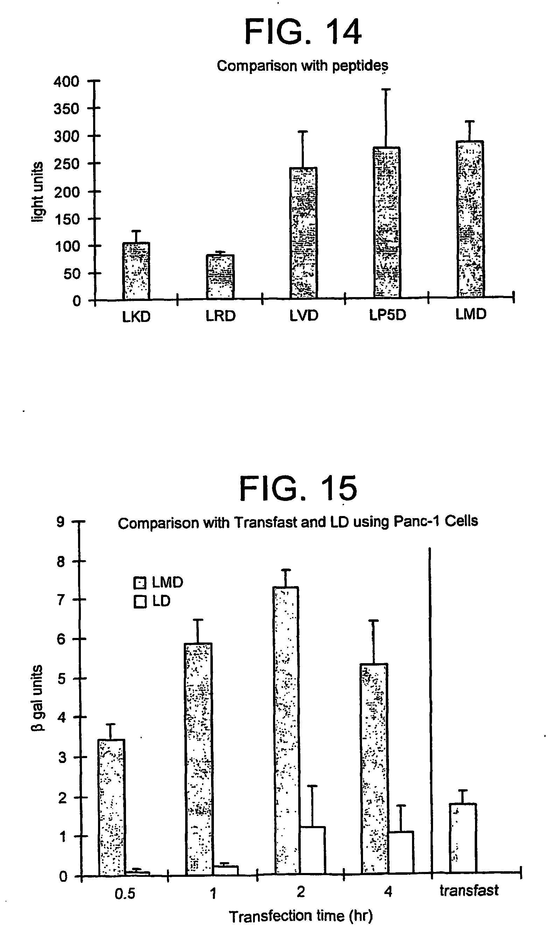

As shown in Fig. 14, the cationic peptides derived from adenovirus (Mu1 and p5) and the Mu1 analogue (V) revealed excellent transfection activity compared to complexes prepared using the synthetic cationic polypeptides, poly lysine and poly arginine.

Amino Acid sequences of p5, V and Mu1 peptide
P5; RPRRRATTRRRTTTGTRRRRRRR
V; VRRVHHRRRRVSHRRVRGG
Mul; MRRAHHRRRRASHRRMRGG

### Example 10 - Comparison with Transfast using Panc-1

LMD and LD were prepared by the same method described in Example 4 except for use of pCMVβ. Transfast (Promega) DNA complex was prepared according to manufacturer's protocol.

Transfection activities were evaluated in vitro using Panc-1 cells. The cells were seeded at an approximate density of 5x10⁴ per well in a 24-well culture plate in RPMI supplemented with 10% FCS and grown for 24h in the presence of 5% CO₂ at 37°C. Cells were washed by brief exposure to RPMI and then treated with solutions containing LMD or LD complexes, prediluted with RPMI (final DNA concentration 5.0µg/ml in all cases), for the times indicated. Cells were then washed again and incubated for a further 48h in RPMI supplemented with 10% FCS prior to harvesting. The level of β-galactosidase enzyme activity was determined with a standard assay kit (Promega). Transfection with Transfast:DNA complex was performed in serum free medium (optimum conditions) for 1h.

As shown in Fig. 15, LMD showed better transfection activity than the Transfast:DNA complex and LD. These results are completely consistent with those found with ND-7 cells.

### Example 11 - Comparison with Lipofectamine using human bronchial cells

The transfection activity of LMD complexes was compared with that of Lipofectamine (Gibco) complexed with DNA using HBE cells (human bronchial epithelium cell).

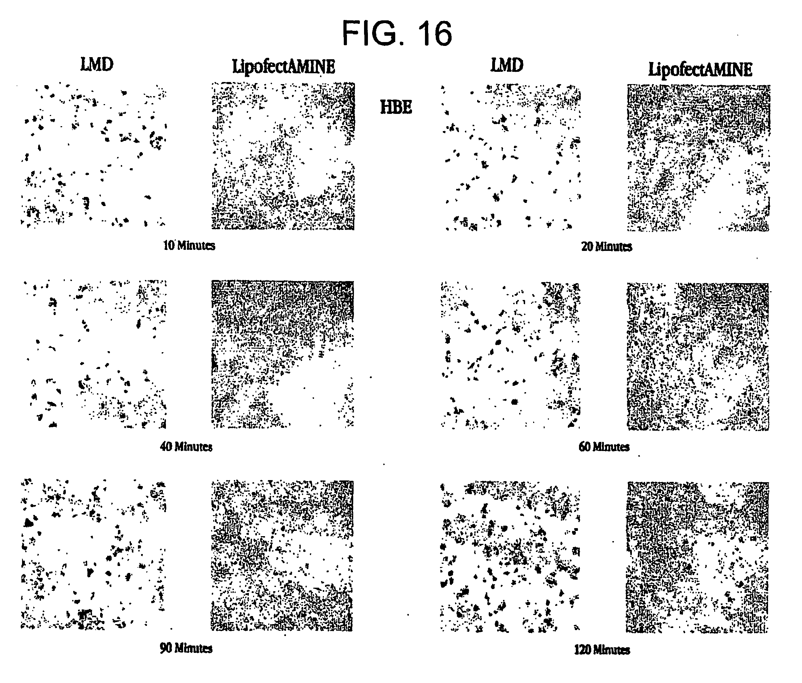

The cells were seeded in a 12-well culture plate in DMEM supplemented with 10% FCS and grown for 24h in the presence of 5% CO₂ at 37°C. Cells were washed by brief exposure to DMEM and then treated with solutions containing either LMD (prepared as in Example 4) or LD (prepared from lipofectamine:DNA 12:1 w/w) complexes, prediluted with OPTIMEM (Gibco) (final DNA concentration 5.0µg/ml in all cases), for the indicated times (see Fig 16). Cells were then washed again and incubated for a further 48h in DMEM supplemented with 10% FCS prior to processing for histochemical staining with X-gal.

LMD showed a better transfection activity than lipofectamine (Fig. 16) and exhibited a more rapid uptake by HBE cells. Similar results were seen with ND7 and Panc-1 cells.

### Example 12 - Comparison with LT1 using rat brain; ex vivo experiment

We assessed transfection activities in organotypic cultures from the rat brain using a reporter DNA (pCMVβ) in order to mimic an in vivo model. Brain slices were maintained on transparent porous membranes and were observed to maintain their intrinsic connectivity and cytoarchitecture to a large degree.

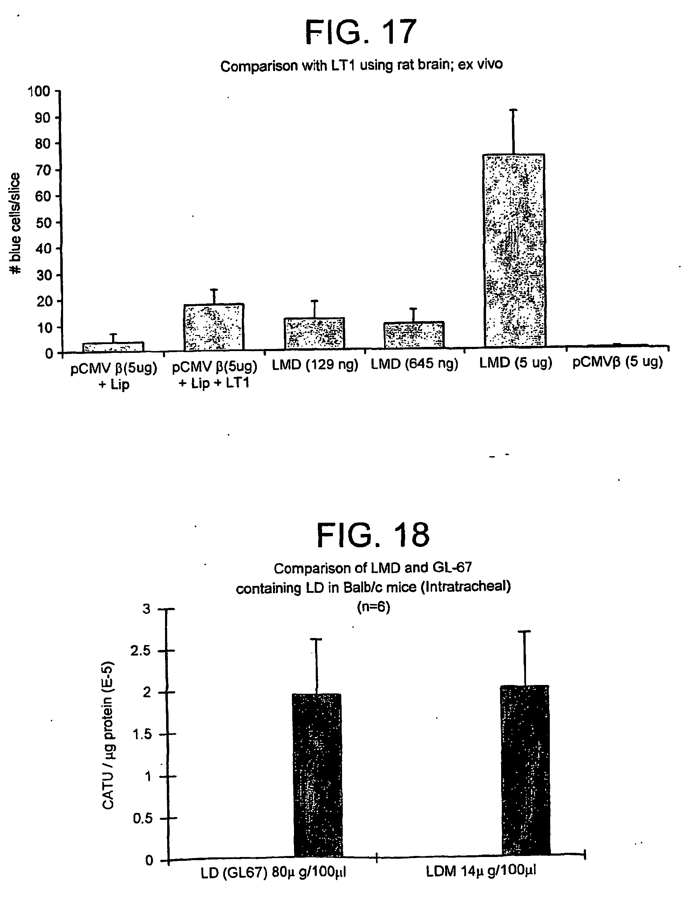

LMD and LD were prepared as shown in Example 4. LT1 is a polyamine transfection reagent manufactured by Pan Vera Co. A complex containing cationic liposome (DC-Chol:DOPE, 3:2 m/m), LT-1 and pCMVβ plasmid in the ratio 3:3.2:1 (w/w/w) was prepared. Brain slices were treated with solutions containing LMD, LD or liposome:LT1:DNA for 2h (Murray *et al., Gene Ther.* 1999, **6**, 190-197). In all cases no morphological changes in the sections were observed during the experiment. After 48h incubation post-transfection, cells were harvested, X-gal stained and the number of blue cells counted on a slice (Fig. 17).

At a DNA dose of 5.0µg (2ml culture), LMD gave an apparently larger number of blue stained cells than LD or LT1 complex after X-gal staining. At a dose as low as 129ng, LMD showed considerable transfection activity, still higher than that of LD (DC-Chol:DOPE complexed to DNA, 3:1 w/w ratio) (DNA dose 5.0µg). We found much higher reporter gene expression with LMD compared to transfection mediated by LD and liposome:LT1:DNA complexes. In fact, LMD mediated transfection was over 19 times more effective than LD and over 4 times more effective than liposome:LT1:DNA at comparable doses.

### Example 13 - Comparison with GL-67 cationic liposomes; in vivo experiment in mouse lung

We assessed the transfection activity in mouse lung in vivo of LMD (prepared as described in Example 4 using DC-Chol:DOPE cationic liposomes [3:2, m/m] and pCF1-CAT plasmid), comparing this with the transfection activity of cationic liposomes GL-67:DOPE:DMPE-PEG₅₀₀₀ (1:2:0.05 m/m/m) complexed with pCF1-CAT plasmid (LD) (liposome:DNA ratio 5.4:1 w/w) used to great effect in lung clinical trials (Alton *et al., Lancet,* 1999, **353**, 947-954).

LMD (final DNA concentration 0.14mg/ml; 100µl volume; DNA dose 14µg) was instilled into the lungs of Balb/c mice. GL-67:DOPE:DMPE-PEG₅₀₀₀ (1:2:0.05 m/m/m) was complexed with pCF1-CAT plasmid (final DNA concentration 0.8mg/ml; 100µl volume; DNA dose 80µg) and this LD complex was similarly instilled into the lungs of Balb/c mice. After 48h, the lungs were homogenised and assayed for CAT activity. Error bars indicate s.e.m.

The results show (Fig. 18) that LMD and the GL-67 containing LD system gave essentially equivalent levels of transfection in vivo even though the LMD system was delivering a five fold lower DNA dose.

### Example 14 - Sugar modified LMD systems

Unspecific interactions of LMD with the biological environment should be minimised for *in vivo* applications. For example, during intravenous administrations undesired interactions with blood components (salts, proteins...) and non-target cells are important obstacles. This opsonization of foreign particles with plasma proteins presents one of the first steps in the natural process of removal of foreign particles by the innate immune system. To reduce proteins binding and salt induced aggregations; naturally occurring polysaccharides can be coupled to LMD. This carbohydrate modification of LMD can be as well applied for targeting of LMD to carbohydrates receptors.

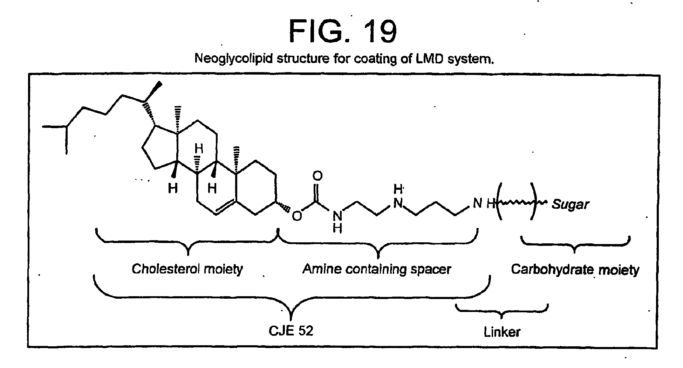

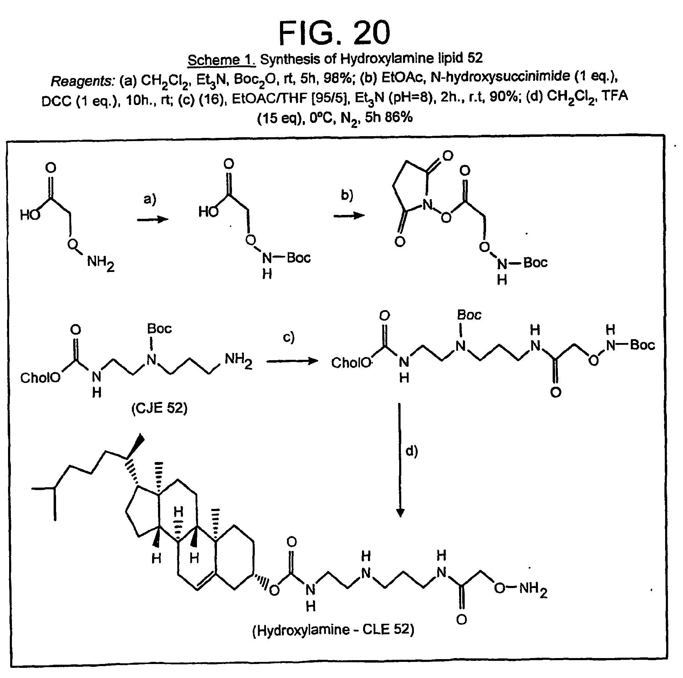

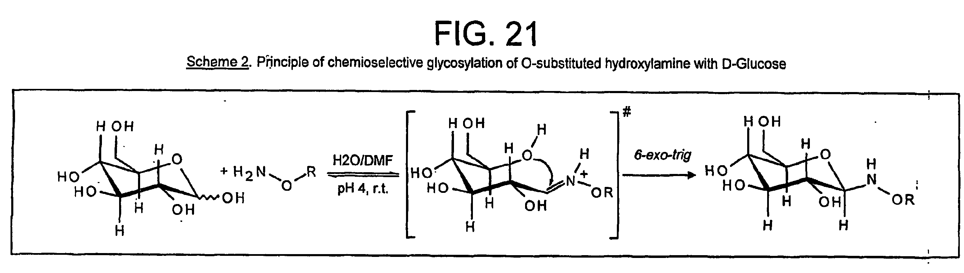

To obtain the desired effect, we designed the neoglycolipids described in Fig. 19. Those compounds are based onto three distinct domains.
ACHx (CJE 52): This lipid (see Fig. 9) was chosen as generic lipid platform for the desired neoglycolipids. The cholesterol aliphatic ring system represents a very hydrophobic area that inserts inside the lipid coat of LMD or LD particles acting as a neoglycolipid anchor. Carbohydrate motif: The choice of oligosaccharides was limited by the complexity of any chemistry involving carbohydrate modifications. We decided to use the long chain commercially available carbohydrates maltotetraose and maltohepataose as proof of principle.
Linker: Use of a chemoselective linkage proved efficient and flexible, allowing us to synthesise a wide range of neoglycolipids. This chemoselective technique was based upon a conversion of CJE52 into an hydroxylamino lipid that was able to couple directly to unprotected carbohydrates. The synthesis of a typical hydroxylamino-CJE52 is shown in Figure 20 - Scheme 1 and the coupling of the carbohydrate moiety onto the linker is based on the glycosylation of an O-substituted hydroxylamine (The principle of the reaction with Glucose is illustrated in Figure 21 - Scheme 2). Following this strategy, Maltotetraose and Maltoheptaose were coupled to obtain GLU4 and GLU7 compounds (Structure in Fig. 22).

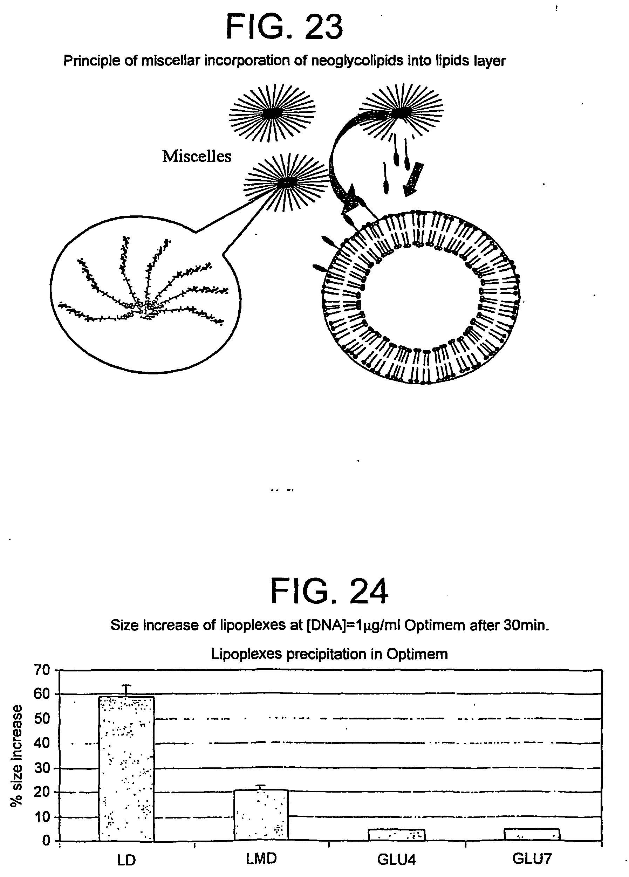

The glyco-modification of LMD was based on the natural ability of neoglycolipid micelles to dissociate and free lipids incorporate into LMD membranes. Firstly LMD were formulated from DC-Chol:DOPE cationic liposomes, Mu1 peptide and pCMVβ plasmid as described in Example 4. Thereafter, a suspension of neoglycolipid micelles in Hepes Buffer, pH 7.0 was added to LMD mixtures and the whole incubated for 30 min at 20°C before storage at -80°C (Fig. 23).

Neoglycolipids Stabilisation of LMD: the stabilisation effect of neoglycolipid modified LMD was evaluated by incorporation of 7.5mol% of GLU4 or GLU7 into LMD. The lipid layer of LD systems is known to aggregate after salt exposure. Therefore, the sizes of LD (final DNA concentration 1µg/ml) particles were evaluated after 30min at 37°C in OPTIMEM by Photon Correlation Spectroscopy (N4 plus, Coulter). Unimodal analysis was used to evaluate the mean particle size. The average percentage increase in LD particle size is shown (Fig. 24). The same procedure was followed for the basic LMD system, LMD(GLU4) and LMD(GLU7) (final DNA concentrations 1µg/ml).

The results indicate that LMD is more stable than LD in solution but also show that the presence of GLU4 and GLU7 has an enhanced anti-aggregation stabilising effect on LMD particles at 7.5mol%.

In vitro transfection efficiency: transfection activity was determined with Hela cells seeded at 5x10⁴ cells per well in 24-well culture plates and grown to approximately 70% confluence in DMEM supplemented with FCS at 37°C and in the presence, of 5% CO₂. Cells were washed in PBS and then treated with solutions containing LMD complexes, prediluted with DMEM containing FCS at the indicated percentages (%) (final DNA concentration 5.0µg/ml in all cases), for 30min. Cells were further washed and then incubated for a further 48h in normal medium (NGM) prior to harvesting. The level of β-galactosidase expression was determined with a standard assay kit (chemiluminescent, Roche).

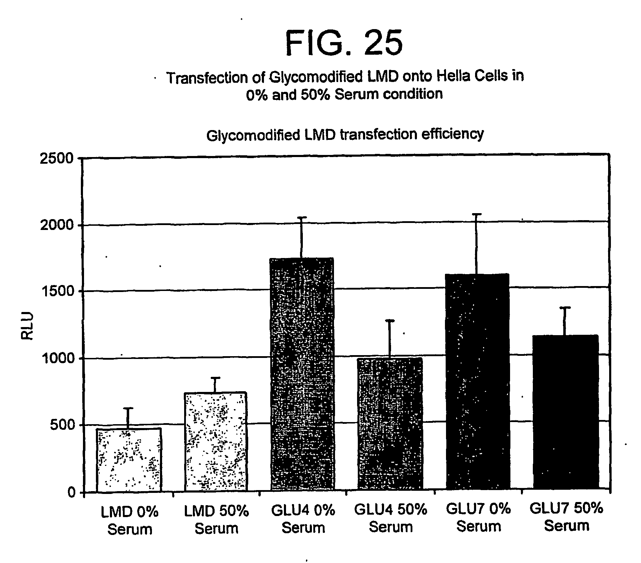

The results indicate an enhancement of the transfection efficiency due to Sugar modification in both 0% and 50% Serum conditions (Fig. 25).

### Discussion

We have previously shown that DC-Chol/DOPE liposomes are efficient at transfecting the neuronally derived ND7 cell line ³¹. DC-Chol has been used successfully outside the CNS in a variety of tissues and has undergone clinical trials for gene therapy treatments of cystic fibrosis ³³, ³⁴. Also, DC-Chol liposomes have been shown not to exhibit cytotoxic side effects ^{35, 36}. For these reasons we wish to develop improved formulations of these liposomes for use in neural cells.

We describe here the use of a virus-coded protein for cellular transfection. We found that Mu1, when used in combination with the cationic liposome DC-Chol/DOPE was able to improve significantly cellular transfection. This effect was most likely due to the ability of Mu1 to condense pDNA and could be optimized by varying the ratios of polypeptide, pDNA and cationic liposome. Significantly, the enhancement in transfection efficiency was more pronounced on differentiated cells. As mentioned above, ND7 cells were derived from primary DRGs. Differentiating ND7 cells induces a phenotype similar to their parental peripheral sensory neurons including the induction of neurite outgrowth, a reduction in overall proliferation and a reduction in transfectability ^{28, 37}. An enhancement in transfection efficiency in differentiated ND7s may reflect an enhanced ability to promote transfections in primary neurons or *in vivo.*

The success of non-viral gene delivery vehicles as viable alternatives to virus vector-based systems is dependent on the development of complexes with higher and longer lasting transfection efficiencies. Since the initial identification of cationic liposomes as vehicles for the transfer of genetic material into cells there has been a large push to develop better cationic liposome formulations ^{5, 7}. Most attempts at improving cationic liposomes have been based on structural modifications to the molecule itself ³⁰. Novel formulations have been developed which have improved transfection efficiencies ³⁰. However, particular cell types behave differently in regards to cationic liposomal transfection. For example, we found the polypeptide Mu1 better at enhancing cationic liposome mediated transfection than Vp1. This was probably due to Mul's greater charge ratio. While both peptides are approximately the same molecular weight, the overall charge ratio of Mu1 was more than twice that of Vp1 (Table 1). Consistent with this Mu1 was able to retard the electrophoretic mobility of plasmid DNA at less than 1/60^{th} the concentration demonstrating how tightly Mu1 is able to bind DNA. While a small shift in pDNA mobility was detected when 0.25 µg Mu1 was complexed to 1 µg pCMVβ, almost all of the plasmid was retained near the loading well following addition of 0.5 µg Mu1 (Figure 1). A 0.5/1.0 (w/w) ratio of Mu1 to pCMVβ corresponds to a 1000/1 molar ratio. Each molecule of Mu1 contains 12 residues that could potentially carry a positive charge. The theoretical charge ratio of Mu1 to pCMVβ would then be 1.6 (12000 Mu1 cations to 7500 pCMVβ anions). This ratio should completely neutralize the negative charges on pCMVβ thus completely retarding its migration as seen.

A direct comparison between the amount of Mu1 that significantly retarded plasmid DNA migration and that which optimally enhanced transfections could not be made since the method of preparation was different. The peptide-pDNA-liposome transfection complexes were prepared in larger volumes (see Materials and Methods). Although it took 24 times as much Mu1 (12 µg /1 µg pCMVβ) to achieve optimal enhancement of transfection efficiencies as it did to retard migration in an agarose gel, the concentration in solution was similar (25ng/mL, pDNA retardation; 30ng/mL, optimal transfections). The presence of Mu1 also altered cationic liposome pDNA interactions. The optimal ratio of DC-Chol/DOPE to pCMVβ in the presence of Mu1 was 6/1, twice that previously found optimal in neuronal cells ^{31, 38}. Theoretically the amount of Mu1 used should have completely neutralized the positive charges on pCMVβ, which would have prevented further complexing with DC-Chol/DOPE. Clearly this was not the case since much improved transfection efficiencies were attainable. It's likely that not all the possible charged amino acids were protonated in our buffer conditions. Why more cationic liposomes are required to improve transfections is not clear and we are currently working to address this question.

Finally a point should be made regarding the nuclear localization signal embedded within Vp1. Recent evidence in our laboratory (unpublished observations) and in others ^{10, 11}, ^{39, 40} has suggested that nuclear transport of transfected material may be inefficient in lipofection. For this reason attempts have been made to pre-condense DNA with polycations containing peptide sequences known to have nuclear localizing capabilities with the aim of improving nuclear uptake of transfected DNA ^{17, 20, 22}. We found however, that the more efficient DNA condensing properties of Mu1 far outweighed the nuclear localizing capacity of Vp1 in terms of improving transfection efficiencies. Similarly Fritz et al., ²² found no difference in transfection efficiencies between recombinant human histone (H1) and a modified version containing the SV40 large T antigen nuclear localizing sequence. Other studies have suggested that the presence of an NLS does improve nuclear accumulation of transfected pDNA albeit via specific intracellular pathways ^{41, 42}.

### References

1. Wood MJA *et al.* Inflammatory effects of gene-transfer into the CNS with defective HSV-1 vectors. *Gene Ther* 1994; **1**: 283-291.
2. Byrnes AP *et al.* Adenovirus gene-transfer causes inflammation in the brain. *Neuroscience* 1995; **66**: 1015-1024.
3. Naldini L *et al.* In vivo gene delivery and stable transduction of nondividing cells by a lentiviral vector [see comments]. *Science* 1996; **272**: 263-267.
4. Miller AD. Cationic liposomes for gene delivery. *Angewandte Chemie-International Edition* 1998; **37**: 1769-1785.
5. Lee RJ, Huang L. Lipidic vector systems for gene transfer. *Critical Reviews in Therapeutic Drug Carrier Systems* 1997; **14**: 173-206.
6. Gao X, Huang L. Cationic liposome-mediated gene-transfer. *Gene Ther* 1995; **2**: 710-722.
7. Felgner PL *et al.* Lipofection - a Highly Efficient, Lipid-Mediated Dna-Transfection Procedure. *Proceedings Of the National Academy Of Sciences Of the United States Of America* 1987; **84**: 7413-7417.
8. Farhood H, Serbina N, Huang L. The role of dioleoyl phosphatidylethanolamine in cationic liposome mediated gene transfer. *Biochim Biophys Acta* 1995; **1235**: 289-295.
9. Caplen NJ *et al.* In-vitro liposome-mediated DNA transfection of epithelial-cell lines using the cationic liposome DC-Chol/DOPE. *Gene Ther* 1995; **2**: 603-613.
10. Labat-Moleur F *et al.* An electron microscopy study into the mechanism of gene transfer with lipopolyamines. *Gene Ther* 1996; **3**: 1010-1017.
11. Zabner J *et al.* Cellular and molecular barriers to gene transfer by a cationic lipid *J Biol Chem* 1995; **270**: 18997-19007.
12. Felgner JH *et al.* Enhanced Gene Delivery and Mechanism Studies With a Novel Series Of Cationic Lipid Formulations. *J Biol Chem* 1994; **269**: 2550-2561.
13. Sahenk Z *et al.* Gene Delivery to Spinal Motor Neurons. *Brain Res* 1993; **606**: 126-129.
14. Iwamoto Y *et al.* Liposome-Mediated Bdnf Cdna Transfer In Intact and Injured Rat-Brain. *Neuroreport* 1996; **7**: 609-612.
15. Roessler BJ, Davidson BL. Direct plasmid-mediated transfection of adult murine brain-cells *in-vivo* using cationic liposomes. *Neurosci Lett* 1994; **167**: 5-10.
16. Zhou X, Huang L. DNA transfection mediated by cationic liposomes containing lipopolylysine: characterization and mechanism of action. *Biochim Biophys Acta* 1994; **1189**: 195-203.
17. Sorgi FL, Bhattacharya S, Huang L. Protamine sulfate enhances lipid-mediated gene transfer. *Gene Ther* 1997; **4**: 961-968.
18. Li S, Huang L. Protamine sulfate provides enhanced and reproducible intravenous gene transfer by cationic liposome/DNA complex *Journal of Liposome Research* 1997; **7**: 207-219.
19. Vitiello L *et al.* Condensation of plasmid DNA with polylysine improves liposome-mediated gene transfer into established and primary muscle cells. *Gene Ther* 1996; **3**: 396-404.
20. Namiki Y, Takahashi T, Ohno T. Gene transduction for disseminated intraperitoneal tumor using cationic liposomes containing non-histone chromatin proteins: cationic liposomal gene therapy of carcinomatosa *Gene Ther* 1998; **5**: 240-246.
21. Gao X, Huang L. Potentiation of cationic liposome-mediated gene delivery by polycations. *Biochem* 1996; **35**: 9286-9286.
22. Fritz JD *et al.* Gene transfer into mammalian cells using histone-condensed plasmid DNA. *Hum Gene Ther* 1996; **7**: 1395-1404.
23. Hagstrom JE *et al.* Complexes of non-cationic liposomes and histone H1 mediate efficient transfection of DNA without encapsulation. *Biochim Biophys Acta* 1996; **1284**: 47-55.
24. Isaka Y *et al.* The HVJ liposome method *Exp-Nephrol* 1998; **6**: 144-147.
25. Gillock ET *et al.* Polyomavirus major capsid protein VP1 is capable of packaging cellular DNA when expressed in the baculovirus system. *J-Virol* 1997; **71**: 2857-2865 issn: 0022-2538x.
26. Chang D, Cai X, Consigli RA. Characterization of the DNA binding properties of polyomavirus capsid proteins. *Journal of Virology* 1993; **67**: 6327-6331.
27. Anderson CW, Young ME, Flint SJ. Characterization of the adenovirus 2 virion protein, mu. *Virology* 1989; **172**: 506-512.
28. Wood JN *et al.* Novel Cell-Lines Display Properties of Nociceptive Sensory Neurons. *Proceedings of the Royal Society of London Series B-Biological Sciences* 1990; **241**: 187-194.
29. Budhrammahadeo V, Lillycrop KA, Latchman DS. The Levels of the Antagonistic Pou Family Transcription Factors Brn- 3a and Bm-3b in Neuronal Cells Are Regulated in Opposite Directions By Serum Growth-Factors. *Neurosci Lett* 1995; **185**: 48-51.
30. Cooper RG *et al.* Polyamine analogues of 3 beta-[N-(N',N'-dimethylaminoethane)carbomoyl]cholesterol (DC-Chol) as agents for gene delivery. *Chemistry-a European Journal* 1998; **4**: 137-151.
31. McQuillin A *et al.* Optimization of liposome mediated transfection of a neuronal cell line. *Neuroreport* 1997; **8**: 1481-1484.
32. Hosokawa K, Sung MT. Isolation and characterization of an extremely basic protein from adenovirus type 5. *Journal Of Virology* 1976; **17**: 924-934..
33. Caplen NJ *et al.* Liposome-Mediated Cftr Gene-Transfer to the Nasal Epithelium of Patients With Cystic-Fibrosis. *Nature Med* 1995; **1**: 39-46.
34. Nabel G, Chang A, Nabel E. Clinical, Protocol: Immunotherapy of malignancy by *in vivo* gene transfer into tumors. *Hum Gene Ther* 1992; **3**: 399-410.
35. Nabel GJ *et al.* Direct gene-transfer with DNA liposome complexes in melanoma-expession, biological activity, and lack of toxicity in humans. *Proc Natl Acad Sci USA* 1993; **90**:11307-11311.
36. Stewart MJ *et al.* Gene-transfer in vivo with DNA liposome complexes - safety and acute toxicity in mice. *Hum Gene Ther* 1992; **3**: 267-275.
37. Murray KD *et al.* DC-Chol/DOPE mediated transfections in differentiated sensory neurons. *In preparation* 1999*.*
38. Murray KD *et al.* Cationic liposome-mediated transfection in organotypic explant cultures. *Gene Ther* 1999; **6**: 190-197.
39. Thierry AR *et al.* Characterization of liposome-mediated gene delivery: Expression, stability and pharmacokinetics of plasmid DNA *Gene Ther* 1997; **4**: 226-237.
40. Coonrod A, Li FQ, Horwitz M. On the mechanism of DNA transfection: efficient gene transfer without viruses. *Gene Ther* 1997; **4**: 1313-1321.
41. Sebestyen MG *et al.* DNA vector chemistry: the covalent attachment of signal peptides to plasmid DNA *Nat Biotechnol* 1998; **16**: 80-85.
42. Hagstrom JE *et al.* Nuclear import of DNA in digitonin-permeabilized cells. *J Cell Sci* 1997; **110 (Pt 18)**: 2323-2331:

## Claims

1. A non-viral nucleic acid delivery vector comprising a condensed polypeptide/nucleic acid complex and a cationic lipid, wherein the complex comprises
(a) a nucleic acid sequence of interest (NOI); and
(b) one or more Mu1 Adenoviral polypeptides or homologous polypeptides with at least 60 % identity thereto, said polypeptides or said homologues thereof being (i) capable of binding to the NOI; and (ii) capable of condensing the NOI; and wherein the NOI is heterologous to the one or more polypeptides.

2. A vector according to claim 1 further comprising a polypeptide comprising a nuclear localisation sequence (NLS).

3. A vector according to claim 2 wherein the polypeptide comprising a nuclear localisation sequence (NLS) is adenoviral pV or a derivative thereof.

4. A condensed polypeptide/nucleic acid complex comprising a cationic lipid, a polypeptide component and a nucleic acid component, for use in delivering the nucleic acid component to a nucleus of a eukaryotic cell, wherein
(a) the polypeptide component is a Mu1 Adenoviral polypeptide or homologous polypeptide with at least 60 % identity thereto;
(b) the polypeptide component or said homologue thereof is capable of binding to the NOI; and
(c) the polypeptide component or said homologue thereof is capable of condensing the NOI;
and wherein the nucleic acid is heterologous to the polypeptide.

5. A complex according to claim 4 further comprising a polypeptide comprising a nuclear localisation sequence (NLS).

6. A complex according to claim 5 wherein the polypeptide comprising a nuclear localisation sequence (NLS) is adenoviral pV or a derivative thereof.

7. A complex according to any of claims 4 to 6, wherein the ratio of liposome:NOI:polypeptide is 2-20:1:0.5-1, preferably 10-14:1:0.5-0.7, more preferably approximately 12:1:0.6.

8. A complex according to any of claims 4 to 7, wherein the complex is under 200 nm in diameter.

9. A method of producing a non-viral nucleic acid delivery vector comprising a cationic lipid and a condensed polypeptide/nucleic acid complex, which method comprises:
(a) contacting a nucleic acid sequence of interest (NOI) with a Mu1 polypeptide or a homologous polypeptide with at least 60 % identity thereto, said polypeptide component or said homologue thereof being (i) capable of binding to the NOI; and (ii) capable of condensing the NOI; and wherein the NOI is heterologous to the polypeptide; and
(b) contacting the nucleic acid/polypeptide complex thus formed with a cationic lipid.

10. A method of introducing a nucleic acid sequence of interest (NOI) into a eukaryotic cell which method comprises contacting the cell in vitro with a complex according to any of claims 4 to 8, wherein the complex comprises the NOI.

11. A method according to claim 10 wherein the cell is a neuronal cell, cancer cell, or epithelial cell.

12. Use of a Mu1 Adenoviral polypeptide or a homologous polypeptide with at least 60 % identity thereto in the manufacture of a nucleic acid delivery vector as defined in any of claims 1 to 3.

## Patentansprüche

1. Nicht-viraler Nukleinsäure-Auslieferungsvektor, welcher einen kondensierten Polypeptid-/Nukleinsäure-Komplex und ein kationisches Lipid umfaßt, wobei der Komplex folgendes aufweist:
(a) eine interessierende Nukleinsäuresequenz (NOI) und
(b) ein oder mehrere Adenovirus-Mu1-Polypeptide oder homologe Polypeptide mit wenigstens 60% Identität hierzu, wobei die Polypeptide oder deren Homologe (i) in der Lage sind, an die NOI zu binden, und (ii) in der Lage sind, die NOI zu kondensieren, und wobei die NOI heterolog zu dem einen oder den mehreren Polypeptiden ist.

2. Vektor nach Anspruch 1, welcher weiterhin ein Polypeptid umfaßt, das eine Kemlokalisierungssequenz (NLS) enthält.

3. Vektor nach Anspruch 2, wobei das Polypeptid, das eine Kemlokalisierungssequenz (NLS) enthält, Adenovirus-pV oder ein Derivat davon ist.

4. Kondensierter Polypeptid-/Nukleinsäure-Komplex, welcher ein kationisches Lipid, eine Polypeptidkomponente und eine Nukleinsäurekomponente umfaßt, zur Verwendung bei der Auslieferung der Nukleinsäurekomponente an einen Zellkem einer eukaryontischen Zelle, wobei
(a) die Polypeptikomponente ein Adenovirus-Mu1-Polypeptid oder ein homologes Polypeptid mit wenigstens 60% Identität hierzu ist,
(b) die Polypeptidkomponente oder deren Homologes in der Lage ist an die NOI zu binden, und
(c) die Polypeptidkomponente oder deren Homologes in der Lage ist, die NOI zu kondensieren,
und wobei die Nukleinsäure heterolog zu dem Polypeptid ist.

5. Komplex nach Anspruch 4, welcher weiterhin ein Polypeptid umfaßt, das eine Kemlokalisierungssequenz (NLS) enthält.

6. Komplex nach Anspruch 5, wobei das Polypeptid, das eine Kemlokalisierungssequenz (NLS) enthält, Adenovirus-pV oder ein Derivat davon ist.

7. Komplex nach einem der Ansprüche 4 bis 6, wobei das Verhältnis von Liposom:NOI:Polypeptid 2-20:1:0,5-1, bevorzugt 10-14:1:0,5-0,7 und ganz besonders bevorzugt etwa 12:1:0,6 beträgt.

8. Komplex nach einem der Ansprüche 4 bis 7, wobei der Komplex einen Durchmesser von weniger als 200 nm hat.

9. Verfahren zum Herstellen eines nicht-viralen Nukleinsäure-Auslieferungsvektors, welcher ein kationisches Lipid und einen kondensierten Polypeptid-/Nukleinsäure-Komplex umfaßt, wobei das Verfahren folgendes umfaßt:
(a) Inkontaktbringen einer interessierenden Nukleinsäuresequenz (NOI) mit einem Mu1-Polypeptid oder einem homologen Polypeptid mit wenigstens 60% Identität hierzu, wobei die Polypeptidkomponente oder deren Homologes (i) in der Lage sind, an die NOI zu binden, und (ii) in der Lage sind, die NOI zu kondensieren, und wobei die NOI heterolog zu dem Polypeptid ist, und
(b) Inkontaktbringen des so gebildeten Nukleinsäure-/Polypeptid-Komplexes mit einem kationischen Lipid.

10. Verfahren zum Einführen einer interessierenden Nukleinsäuresequenz (NOI) in eine eukaryontische Zelle, wobei das Verfahren das Inkontaktbringen der Zelle in vitro mit einem Komplex gemäß einem der Ansprüche 4 bis 8 umfaßt und wobei der Komplex die NOI umfaßt.

11. Verfahren nach Anspruch 10, wobei die Zeile eine Neuronenzelle, eine Krebszeile oder eine Epithelzeile ist.

12. Verwendung eines Adenovirus-Mu1-Polypeptids oder eines homologen Polypeptids mit wenigstens 60% Identität hierzu bei der Herstellung eines Nukleinsäure-Auslieferungsvektors, wie in einem der Ansprüche 1 bis 3 definiert.

## Revendications

1. Vecteur d'administration d'acide nucléique non viral, comprenant un complexe polypeptide/acide nucléique condensé et un lipide cationique, dans lequel le complexe comprend
(a) une séquence d'acide nucléique intéressante (NOI) ; et
(b) un ou plusieurs polypeptides adénoviraux Mu1 ou polypeptides homologues ayant une identité d'au moins 60 % avec ceux-ci, lesdits polypeptides ou lesdits homologues de ceux-ci étant (i) capables de se lier à la NOI ; et (ii) capables de condenser la NOI ; la NOI étant hétérologue vis-à-vis du ou des polypeptides.

2. Vecteur suivant la revendication 1, comprenant un polypeptide comprenant une séquence à localisation nucléaire (NLS).

3. Vecteur suivant la revendication 2, dans lequel le polypeptide comprenant une séquence à localisation nucléaire (NLS) est le pV adénoviral ou un de ses dérivés.

4. Complexe polypeptide/acide nucléique condensé comprenant un lipide cationique, un constituant polypeptidique et un constituant du type acide nucléique, destiné à être utilisé pour délivrer le constituant dudit acide nucléique à un noyau d'une cellule eucaryotique, dans lequel
(a) le constituant polypeptidique est un polypeptide adénoviral Mu1 ou un polypeptide homologue présentant une identité d'au moins 60 % avec celui-ci ;
(b) le constituant polypeptidique ou ledit homologue de celui-ci est capable de se lier à la NOI ; et
(c) le constituant polypeptidique ou ledit homologue de celui-ci est capable de condenser la NOI ;
et dans lequel l'acide nucléique est hétérologue vis-à-vis du polypeptide.

5. Complexe suivant la revendication 4, comprenant en outre un polypeptide comprend comprenant une séquence à localisation nucléaire (NLS).

6. Complexe suivant la revendication 5, dans lequel le polypeptide comprenant une séquence à localisation nucléaire (NLS) est le polypeptide adénoviral ou un de ses dérivés.

7. Complexe suivant l'une quelconque des revendications 4 à 6, dans lequel le rapport liposome:NOI:polypeptides est égal à 2-20 :1:0,5-1, avantageusement 10-14:1:05-07, plus avantageusement approximativement 12:1:0,6.

8. Complexe suivant l'une quelconque des revendications 4 à 7, ledit complexe ayant un diamètre inférieur à 200 nm.

9. Procédé pour la production d'un vecteur d'administration d'acide nucléique non viral, comprenant un lipide cationique et un complexe polypeptide/acide nucléique condensé, procédé qui comprend :
(a) la mise en contact d'une séquence d'acide nucléique intéressante (NOI) avec un polypeptide Mu1 ou un polypeptide homologue présentant une identité d'au moins 60 % avec celui-ci, ledit constituant polypeptidique ou ledit homologue de celui-ci étant (i) capable de se lier à la NOI et (ii) capable de condenser la NOI ; la NOI étant hétérologue vis-à-vis du polypeptide ; et
(b) la mise en contact du complexe acide nucléique/polypeptide ainsi formé avec un lipide cationique.

10. Procédé pour l'introduction d'une séquence d'acide nucléique intéressante NOI dans une cellule eucaryotique, procédé qui comprend la mise en contact de la cellule in vitro avec un complexe suivant l'une quelconque des revendications 4 à 8, dans lequel le complexe comprend la NOI.

11. Procédé suivant la revendication 10, dans lequel la cellule est une cellule neuronale ou une cellule cancéreuse ou une cellule épithéliale.

12. Utilisation d'un polypeptide adénoviral Mu1 ou d'un polypeptide homologue présentant une identité d'au moins 60 % avec celui-ci dans la production d'un vecteur d'administration d'acide nucléique tel que défini dans l'une quelconque des revendications 1 à 3.
